# EUROPEAN PATENT SPECIFICATION

(11) **EP 0 516 200 B1**
(45) Date of publication and mention of the grant of the patent: **24.07.1996**
(21) Application number: 92201150.7
(22) Date of filing: 23.04.1992
(51) Int. Cl.: C11D 3/386, C12N 9/52, C12N 9/56

(54) **Detergent compositions containing stabilized enzymes**
Stabilisierte Enzyme enthaltende Waschmittelzusammensetzungen
Compositions de détergents contenant des enzymes stabilisés

(30) Priority: 01.05.1991 EP 91201039
(43) Date of publication of application: 02.12.1992
(73) Proprietor: UNILEVER N.V., NL-3013 AL Rotterdam (NL); UNILEVER PLC, London EC4P 4BQ (GB)
(72) Inventor: Casteleijn, Eric, Unilever Research, NL-3133 AT Vlaardingen (NL); Egmond, Maarten Robert, Unilever Research, NL-3133 AT Vlaardingen (NL); Svendsen, Allen, DK-3460 Birkerod (DK); Von Der Osten, Claus, DK-2800 Lyngby (DK); Hedegard, Lisbeth, DK-2100 Copenhagen 0 (DK); Eriksen, Nina, DK-2000 Frederiksberg (DK); Branner, Sven, DK-2800 Lyngby (DK)
(74) Representative: Kan, Jacob Hendrik, Dr.

(56) References cited:
- WO-A-87/05050
- WO-A-89/09819
- WO-A-91/00345

## Description

### TECHNICAL FIELD

This invention relates to detergent compositions containing noval stabilized protesses.

### BACKGROUND ART

### Proteases/Subtilisins

Proteases, or (interchangeably) peptidases, are enzymes that cleave the amide linkages in protein substrates. Bacteria of the Bacillus species secrete two extracellular species of protease, a neutral or metalloprotease, and an alkaline protease which is functionally a serine endopeptidase, referred to as subtilisin.

A serine protease is an enzyme which catalyses the hydrolysis of peptide bonds, and in which there is an essential serine residue at the active site [*White, Handler and Smith* (1973); Principles of Biochemistry; Fifth Edition, McGraw-Hill Book Company, NY, 271-272].

The bacterial serine proteases have molecular weights in the range of 20,000 to 45,000. They hydrolyse simple terminal esters and are similar in activity to eukaryotic chymotrypsin, also a serine protease. A more narrow term, alkaline protease, covering a sub-group, reflects the high pH optimum of some of the serine proteases, from pH 9.0 to 11.0 [for review, see *Priest;* Bacteriological Rev., **41** 711-753 (1977)].

In relation to the present invention a subtilisin is a serine protease produced by Gram-positive bacteria or fungi. According to another definition, a subtilisin is a serine protease, wherein the relative order of the amino acid residues in the catalytic triad is Asp - His - Ser (positions 32, 64, and 221). A wide variety of subtilisins have been identified, and the amino acid sequences of a number of subtilisins have been determined. These include among others six subtilisins from *Bacillus* strains, namely, subtilisin 168, subtilisin BPN', subtilisin Carlsberg, subtilisin DY, subtilisin amylosacchariticus, and mesentericopeptidase [*Kurihara et al*. (1972); J. Biol. Chem.; **247** 5629-5631; *Wells et al*. (1983); Nucleic Acids Res.; **11** 7911-7925; *Stahl and Ferrari* (1984); J. Bacteriol.; **159** 811-819; *Jacobs et al*. (1985); Nucl. Acids Res.; **13** 8913-8926; *Nedkov et al*. (1985); Biol. Chem. Hoppe-Seyler; **366** 421-430; *Svendsen et al*. (1985); FEBS LETTERS; **196** 228-232] one subtilisin from an actinomycetales, Thermitase from *Thermoactinomyces vulgaris* [*Meloun et al*. (1985); FEBS LETTERS; **1983** 195-200], and one fungal subtilisin, proteinase K from *Tritirachium album* [*Jany and Mayer* (1985); Biol. Chem. Hoppe-Seyler; **366** 584-492].

Proteases such as subtilisins have found much utility in industry, particularly in detergent formulations, as they are useful for removing proteinaceous stains.

### The Structure of Proteins

Proteases are globular proteins and quite compact due to the considerable amount of folding of the long polypeptide chain. The polypeptide chain essentially consists of the "backbone" and its "side-groups". As the peptide bond is planar, only rotations around the C_{α} -N axis and the C_{α}-C' axis are permitted. Rotation around the C_{α}-N bond of the peptide backbone is denoted by the torsion angle φ (phi), rotation around the C_{α}-C' bond by ψ (psi) [*vide* e.g. *Creighton, T.E*. (1984); Proteins; W.H. Freeman and Company, New York]. The choice of the values of these angles of rotation is made by assigning the maximum value of +180° (which is identical to -180°) to the maximally extended chain. In the fully extended polypeptide chain, the N, C_{α} and C' atoms are all "trans" to each other. In the "cis" configuration, the angles φ and ψ are assigned the value of 0°. Rotation from this position around the bonds so that the atoms viewed behind the rotated bond move "counterclockwise" are assigned negative values by definition, those "clockwise" are assigned positive values. Thus, the values of the torsion angles lie within the range -180° to +180°.

Since the C_{α}-atoms are the swivel point for the chain, the side-groups (R-groups) associated with the C_{α}-atoms become extremely important with respect to the conformation of the molecule.

The term "conformation" defines the participation of the secondary and tertiary structures of the polypeptide chains in moulding the overall structure of a protein. The correct conformation of a protein is of prime importance to the specific structure of a protein and contributes greatly to the unique catalytic properties (i.e. activity and specificity) of enzymes and their stability.

The amino acids of polypeptides can be divided into four general groups: nonpolar, uncharged polar, and negatively or positively charged polar amino acids. A protein molecule, when submerged in its aqueous environment in which it normally occurs, tends to expose a maximum number of its polar side-groups to the surrounding environment, while a majority of its nonpolar side groups are oriented internally. Orientation of the side-groups in this manner leads to a stabilization of protein conformation.

Proteins thus exist in a dynamic equilibrium between a folded and ordered state, and an unfolded and disordered state. This equilibrium in part reflects the short range interactions among the different segments of the polypeptide chain, which tend to stabilize the overall structure of proteins. Thermodynamic forces simultaneously tend to promote randomization of the unfolding molecule.

A way to engineer stabilized proteins is to reduce the extent of unfolding by decreasing the flexibility of the polypeptide backbone, and simultaneously decreasing the entropy of the unfolded chain. So far only few attempts have been made to implement this rationale in the development of novel stabilized proteases.

A general principle of increasing protein thermostability has been provided [*Suzuki, Y*. (1989); Proc. Japan Acad.; **65** Ser. B]. In this article Suzuki states that the thermostability of a globular protein can be enhanced cumulatively to a great extent by increasing the frequency of proline occurrence at the second site of β-turns without significant alterations in the secondary and tertiary structures as well as in the catalytic function of enzymes. The principle is based on various facts and findings, among these the fact that proline residues show a strong tendency to occur preferentially at the second site of β-turns [*Levitt, M* (1978); Biochemistry; **17** 4277-4285; and *Chou, P.Y. & Fasman, G.D* (1977); J. Mol. Biol.; **115** 135-175]. The principle is restricted to insertion of proline into the second site of β-turns in proteins, no other sites are mentioned.

International Patent Publication WO 89/01520 (Cetus Corporation, USA) provides a method for increasing the stability of a protein by decreasing the configurational entropy of unfolding the protein. The method is applied on a *Streptomyces rubiqinosus* xylose isomerase, and it involves substitution of an amino acid with proline, or replacement of glycine with alanine, at predicted substitution sites.

In International Patent Publication WO 89/09819 (Genex Corporation, USA) a method for combining mutations for stabilization of subtilisins is provided. This publication lists a number of amino acid mutations that have been found to be thermally stabilizing mutations. The list comprises substitution of serine with proline at position 188 of subtilisins (BPN' numbering).

International Patent Publication WO 87/05050 (Genex Corporation, USA) describes a method for mutagenesis and screening. By this method one or more mutations are introduced by treatment with mutagenizing agents, and the method includes subsequent screening for products with altered properties. As a result of this random mutagenesis a subtilisin with a proline residue at position 188 (BPN' numbering) is provided.

International Patent Publication WO 91/00345 (Novo Nordisk A/S) discloses subtilisin protease variants having improved wash performance, in which the net electrostatic charge has been changed compared to the parent protease. This is achieved by changing the number of (positively or negatively) charged amino acid residues at one or more of in total 157 positions, whereby the subtilisin protease variant has a lower isoelectric point (pIₒ) than that of the parent protease.

It is an object of this invention to provide novel proteases having improved stability.

### SUMMARY OF THE INVENTION

The present invention provides an enzymatic detergent composition comprising a stabilized subtilisin, in which a naturally occurring amino acid residue (other than proline) has been substituted with a proline residue at one or more positions, at which position(s) the dihedral angles φ (phi) and ψ (psi) constitute values within the intervals [-90°<φ<-40° and -180°<ψ<180°], preferably within the intervals [-90°<φ<-40° and 120°<ψ<180°] or [-90°<φ<-40° and -50°<φ<10°], and which position(s) are not located in regions, in which the protease is characterized by possessing α-helical or β-sheet structure, and which substitution is made at one or more of the posititions: 57, 98, 172, 194, 242 and 259 (BPN' numbers).

### Subtilisins

In the context of this invention a subtilisin is defined as a serine protease produced by gram-positive bacteria or fungi. According to another definition, a subtilisin is a serine protease, wherein the relative order of the amino acid residues in the catalytic triad is Asp - His - Ser (positions 32, 64, and 221, BPN' numbering).

### Amino Acids

As abbreviations for amino acids the following symbols are used:

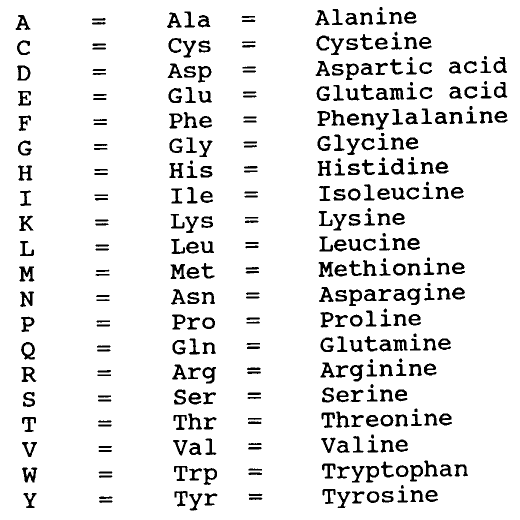

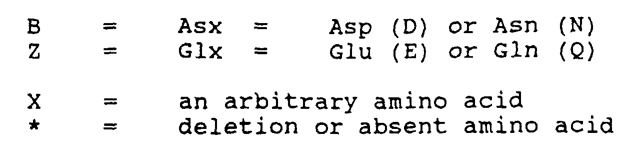

### Protease Variants

A stabilized protease of this invention is a protease variant or mutated protease. By a protease variant or mutated protease is meant a protease obtainable by alteration of a DNA nucleotide sequence of the parent gene or its derivatives. The protease variant or mutated protease may be expressed and produced when the DNA nucleotide sequence encoding the protease is inserted into a suitable vector in a suitable host organism. The host organism is not necessarily identical to the organism from which the parent gene originated.

### Amino Acid Numbering

In the context of this invention a specific numbering of amino acid residue positions in subtilisins is employed. By alignment of the amino acid sequences of various subtilisins along with subtilisin BPN', it is possible to allot a number to the amino acid residue position in any subtilisin to the number of the analogous amino acid position in subtilisin BPN' ("BPN' numbering", *vide* e.g. International Patent Publication Nos. WO 89/06279 and WO 91/00345).

In describing the various protease variants produced or contemplated according to the invention, the following nomenclatures were adapted for ease of reference:

### [Original amino acid; Position; Substituted amino acid]

For example, the substitution of alanine with proline in position 195 is designated as: A195P

Deletion of an aspartic acid at position 36 is indicated as: D36*, and an insertion in such a position is indicated as: *36D for insertion of an aspartic acid in position 36.

Multiple mutations are separated by plusses, i.e.: A194P+G195E representing mutations in positions 194 and 195 substituting alanine with proline and glycine with glutamic acid, respectively.

If a substitution is made by mutation in e.g. subtilisin 309, the product is designated e.g. "subtilisin 309/G195E".

All positions mentioned in this context refer to the BPN' numbers described above.

### Proteolytic Activity

In the context of this invention proteolytic activity is expressed in Kilo NOVO Protease Units (KNPU). The activity is determined relatively to an enzyme standard (SAVINASE™), and the determination is based on the digestion of a dimethyl casein (DMC) solution by the proteolytic enzyme at standard conditions, i.e. 50°C, pH 8.3, 9 min. reaction time, 3 min. measuring time. A folder AF 220/1 is available upon request to Novo Nordisk A/S, Denmark, which folder is hereby included by reference.

### Wash Performance

The ability of an enzyme to catalyse the degradation of various naturally occurring substrates present on the objects to be cleaned during e.g. wash is often referred to as its washing ability, washability, detergency, or wash performance. Throughout this application the term wash performance will be used to encompass this property.

### BRIEF DESCRIPTION OF DRAWINGS

The present invention is further illustrated by reference to the accompanying drawings, in which:
Fig. 1 (sheets 1/14 - 14/14) shows the construction of a synthetic gene; and
Fig. 2 (sheets 1/5 - 5/5) shows the residual activity of subtilisin 309 variants compared to wild type enzyme after storage in a liquid detergent.

### DETAILED DISCLOSURE OF THE INVENTION

The present invention provides an enzymatic detergent composition comprising a stabilized subtilisin in which a naturally occurring amino acid residue (other than proline) has been substituted with a proline residue at one or more positions, at which position(s) the dihedral angles φ (phi) and ψ (psi) constitute values in the intervals [-90°<φ<-40° and -180°<ψ<180°], preferably the intervals [-90°<φ<-40° and 120°<ψ<180°] or [-90°<φ<-40° and -50°<ψ<10°], and which position(s) are not located in regions, in which the protease is characterized by possessing α-helical or β-sheet structure, and which substitution is made at one or more of the positions: 57, 98, 172, 194, 242 and 259 (BPN' numbers).

In the context of this invention, a stabilized protease is a protease variant or mutated protease, being functionally equivalent or having structural features similar to a naturally occurring protease, and in which protease a naturally occurring amino acid residue (other than proline) has been substituted with a proline residue at one or more positions, at which position(s) the dihedral angles φ (phi) and ψ (psi) constitute values within the intervals [-90°<φ<-40° and -180°<ψ<180°], preferably the intervals [-90°<φ<-40° and 120°<ψ<180°] or [-90°<φ<-40° and 50°<ψ<10°], and which position(s) are not located in regions, in which the protease is characterized by possessing α-helical or β-sheet structure.

Moreover, in the context of this invention, a stabilized protease is a protease having improved stability, e.g. in respect to thermal stability, storage stability, etc., when compared to the parent enzyme.

### Defining Secondary Structure of Proteins

The stabilized proteases of the invention may be obtained by subjecting the protease in question to analysis for secondary structure, identifying residues in the protease having dihedral angles φ (phi) and ψ (psi) confined to the intervals [-90°<φ<-40° and -180°<ψ<180°], preferably the intervals [-90°<φ<-40° and 120°<ψ<180°] or [-90°<φ<-40° and -50°<ψ<10°], excluding residues located in regions in which the protease is characterized by possessing α-helical or β-sheet structure, if a proline residue is not already at the identified position(s), substitution of the naturally occurring amino acid residue with a proline residue at the identified position(s), preferably by site directed mutagenesis applied on a gene encoding the protease in question, and gene expression by insertion of the gene encoding the stabilized protease in a suitable host organism, followed by cultivation of said host organism in a suitable nutrient medium, and recovery of the desired protease.

This preparation includes subjecting the protease in question to analysis for secondary structure. To perform such analysis the atomic structure of the protease has to be elucidated. The atomic structure can be determined by X-ray diffraction techniques. X-ray diffraction techniques are described by e.g. *Hendrickson, W.A* [X-ray diffraction; in Protein Engineering (Ed: Oxender, D.L and Fox, C.F.), ch. 1; Alan R. Liss, Inc. (1987)] and *Creighton, T.E*., *supra*, ch.6.

The crystal structure of Subtilisin 309 has been deduced [*vide Betzel, B., Klupsch, S., Papendorf, G., Hastrup, S., Branner, S., and Wilson, K.S*. (1992); J. Mol. Biol. **223** 427-445], and the coordinates have been deposited and are available from the Brookhaven Protein Data Bank [*Bernstein et al*. (1977); J. Mol. Biol. **112** 535-542].

When the atomic structure has been determined, it is possible to compute dihedral angles from the atomic coordinates. Moreover, it is possible to assign secondary structure elements. The secondary structure elements are defined on the basis of hydrogen bindings. Cooperative secondary structure is recognized as repeats of the elementary hydrogen-bonding patterns "turn" and "bridge". Repeating turns are "helices", repeating bridges are "ladders", connected ladders are "sheets".

Analysis for secondary structure elements requires a computerized compilation of structure assignments and geometrical features extracted from atomic coordinates. The conventional method to elucidate the secondary structure of a protein, based on its atomic coordinates, is described by *Kabsch, W. and Sander, C*. [Biopolymers (1983) **22** 2577-2637]. In this article an algorithm for extracting structural features from the atomic coordinates by a pattern-recognition process is provided. First, H-bonds are identified based on electrostatic interactions between pairs of H-bonding groups. Next, the patterns of H-bonding are used to define secondary structure elements such as turns (T), bends (S), bridges (B), helices (G,H,I), β-ladders (E) and β-sheets (E).

A computer program DSSP (Define Secondary Structure of Proteins), enabling the computation of Kabsch & Sander files and written in standard PASCAL, is available from the Protein Data Bank, Chemistry Dept., Brookhaven National Laboratory, Upton, N.Y. 11973.

After the dihedral angles φ (phi) and ψ (psi) for the amino acids have been calculated, based on the atomic structure in the crystalline proteases, it is possible to select position(s) which have dihedral phi and psi angles favourable for substitution with a proline residue. The aliphatic side chain of proline residues is bonded covalently to the nitrogen atom of the peptide group. The resulting cyclic five-membered ring consequently imposes a rigid constraint on the rotation about the N-C_{α} bond of the peptide backbone and simultaneously prevents the formation of hydrogen bonding to the backbone N-atom. For these structural reasons, prolines are generally not compatible with α-helical and β-sheet secondary conformations. Due to the same rotational constraint about the C_{α}-N bond, and due to the requirement that neighbouring amino acids in the chain are not perturbed, the magnitudes of the dihedral angles phi and psi (and in particular phi) are confined to limited intervals for proline residues in polypeptides. The dihedral angles for proline residues in polypeptides are almost exclusively within the intervals [-90°<φ<-40° and -180°<ψ<180°], preferably the intervals [-90°<φ<-40° and 120°<ψ<180°] or [-90°<φ<-40° and -50°<ψ<10°]. In this context both cis- and trans-proline residues are considered.

A proline residue may already occur at one or more positions pointed out by the procedure described above, and then a substitution is, of course, irrelevant. Otherwise the method includes substitution of the naturally occurring amino acid residue with a proline residue.

However, a substitution into proline at every of the predicted positions may not always bring about improved thermostability of the protease. At some of the positions revealed by this method, a substitution of a naturally occurring amino acid residue into a proline residue may even cause destabilisation due to unpredictable factors, such as loss of essential flexibility, loss of H-bond possibilities, unpredictable sterical hindrance, etc. Such "critical sites" are not to be foreseen.

It is to be expected that the stabilizing (or destabilizing) effects of individual substitutions are additive, *vide* e.g. *Wells, J.A*. [Biochemistry (1990) **29** (37) 8510-8517] and Table 4, below.

If a subtilisin different from subtilisin 309 is subjected to this method (although the two subtilisins may seem very much similar), not necessarily the same number of positions, nor particularly the identical positions, may result from the method. It is likely that some of the positions may be identical, and it is likely than the numbers of positions are of equal magnitude, but it is not to be foreseen.

However, it seems likely that the stabilizing proline substitutions resulting from the above described method, applied to any specific protease, also may have a stabilizing effect on any other protease, independent of the result of the above described method applied to such a protease.

When performing the method on a subtilisin 309 molecule (vide International Patent Application No. PCT/DK88/00002), a set of data as listed in Tables 1 and 2 can be obtained. Table 1 depicts a set of positions that - with respect to phi and psi angles - meets one criterion, and Table 2 depicts an additional set of positions that meet another criterion.

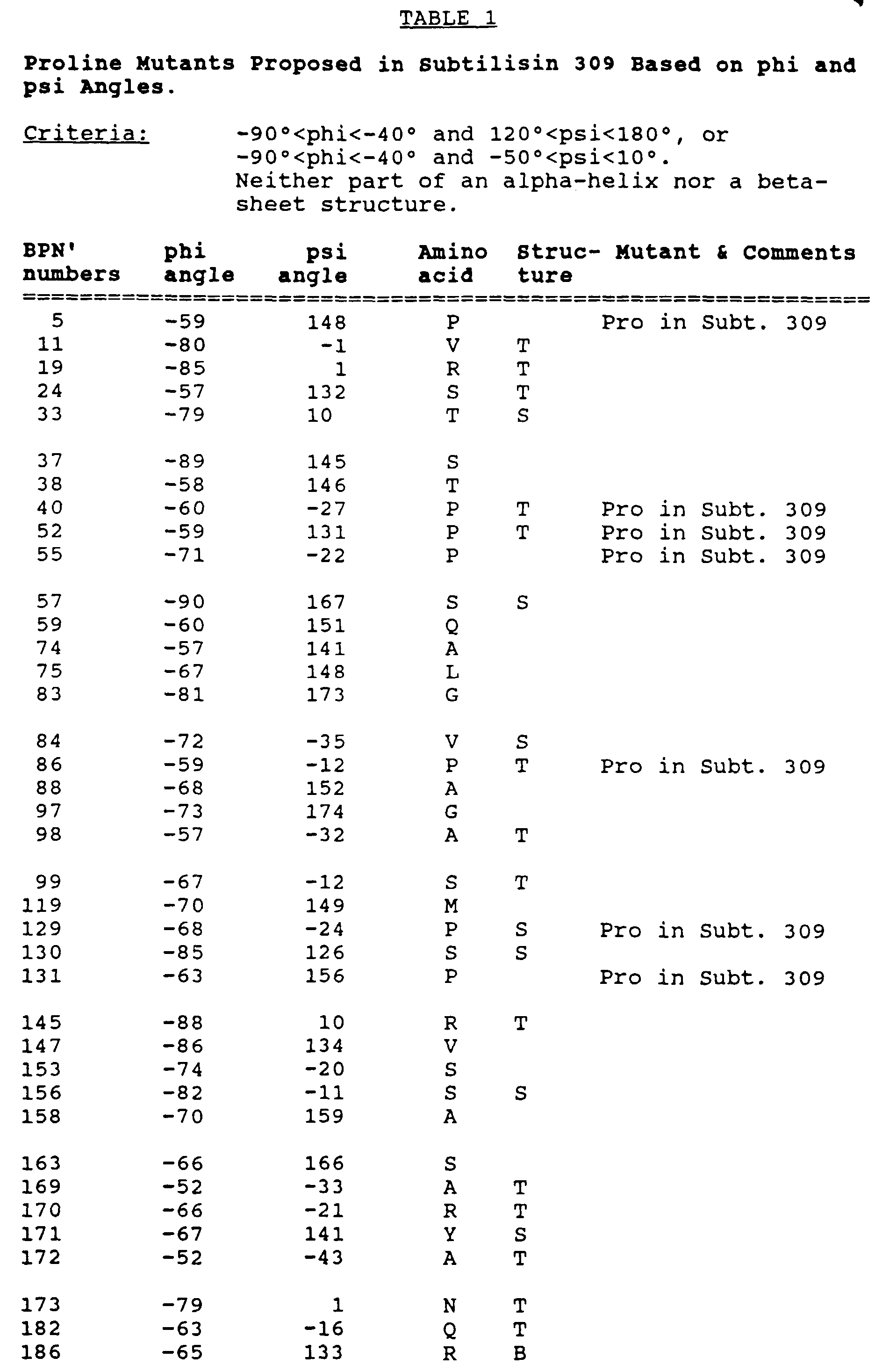

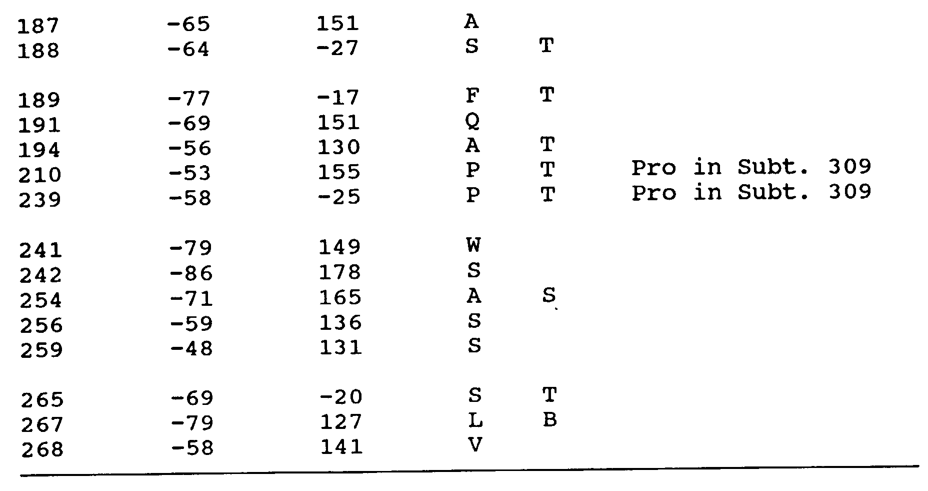

By relaxing the constraint on the psi angle relative to the criteria set up in Table 1, four additional mutants are proposed, *vide* Table 2.

**TABLE 2**

| **Proline Mutants Proposed in Subtilisin 309 based on phi and psi Angles.** | | | | | |
|---|---|---|---|---|---|
| Relaxed criteria : -90°<phi<-40 and -180°<psi<180. Neither part of an alpha helix nor a beta sheet structure. | | | | | |
| **BPN' numbers** | **phi angle** | **psi angle** | **Amino acid** | **Structure** | **Mutant & Comments** |
| 76 | -89 | 102 | N | S | |
| 125 | -90 | 67 | S | | |
| 160 | -83 | 27 | G | S | |
| 255 | -89 | 111 | T | B | |

### Preferred Stabilized Proteases

The protease of the invention is a subtilisin in which a naturally occurring amino acid (other than proline) has been substituted with a proline residue at one or more of the positions: 57, 98, 172, 194, 242, and 259 (BPN' numbers). In further preferred embodiments, the subtilisin in addition comprises one or more of the following substitutions: 27R, 36D, 76D, 97N, 98R, 104Y, 120D, 128G, 195E, 206C, 218S, 235L, 235R, 237R, 251E, and 263F (BPN' numbers).

In a yet more specific aspect the protease of the invention is a stabilized subtilisin 309, a stabilized subtilisin 147, a stabilized subtilisin BPN', or a stabilized subtilisin Carlsberg. Subtilisin 309 and Subtilisin 147 are variants of *Bacillus lentus* and described in US Patent No. 3,723,250 and International Patent Application PCT/DK 88/00002, Subtilisin BPN' is described by *Wells et al.* [Nucleic,Acids Res. (1983) **11** 7911-7925]; Subtilisin Carlsberg is described by Smith et al. [*Smith, E.L.; DeLange, R.J.; Evans, W.H.; Landon, W.; Markland, F.S.* (1968); Subtilisin Carlsberg V. The complete sequence: comparison with subtilisin BPN'; Evolutionary relationships.; J. Biol. Chem. **243** (9) 2184-2191), and *Jacobs et al.* [Nucl. Acids Res. (1985) **13** 8913-8926].

In yet another preferred embodiment the protease of the invention is a subtilisin 309, in which one or more of the following substitutions have been introduced: S57P, A98P, A172P, A194P, S242P, and S259P (BPN' numbers). In a further preferred embodiment, this subtilisin 309 in addition comprises one or more of the following substitutions: K27R, *36D, N76D, G97N, A98R, V104Y, H120D, S128G, G195E, Q206C, N218S, K235L, K235R, K237R, K251E, and Y263F (BPN' numbers).

Most preferred proteases of the invention are: Subtilisin 309/K27R+*36D+G97N+A98R+A194P+K235R+K237R+K251E+Y263F, Subtilisin 309/K27R+*36D+G97N+A194P+K235R+K237R+K251E+Y263F, Subtilisin 309/K27R+*36D+G97N+A194P+K235R+K237R+Y263F, Subtilisin 309/*36D+N76D+H120D+A194P+G195E+K235L, Subtilisin 309/*36D+G97N+V104Y+H120D+A194P+G195E, Subtilisin 309/*36D+G97N+V104Y+H120D+A194P+G195E+K235L, Subtilisin 309/*36D+G97N+H120D+A194P+G195E, Subtilisin 309/*36D+G97N+H120D+A194P+G195E+K235L, Subtilisin 309/*36D+V104Y+H120D+A194P+G195E, Subtilisin 309/*36D+V104Y+H120D+A194P+G195E+K235L, Subtilisin 309/*36D+H120D+A194P+G195E, Subtilisin 309/*36D+H120D+A194P+G195E+K235L and Subtilisin 309/A194P (BPN' numbers).

### The Effect of Proline Stabilization

The purified variants obtained according to this invention have been tested to wash at least equally well compared to the wild-type subtilisin (subtilisin 309), *vide* Example 5 for experimental data.

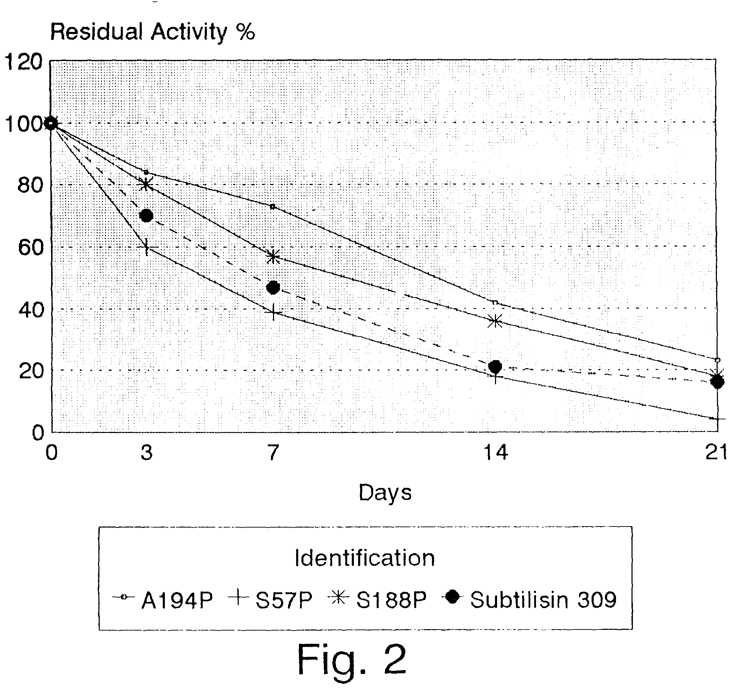

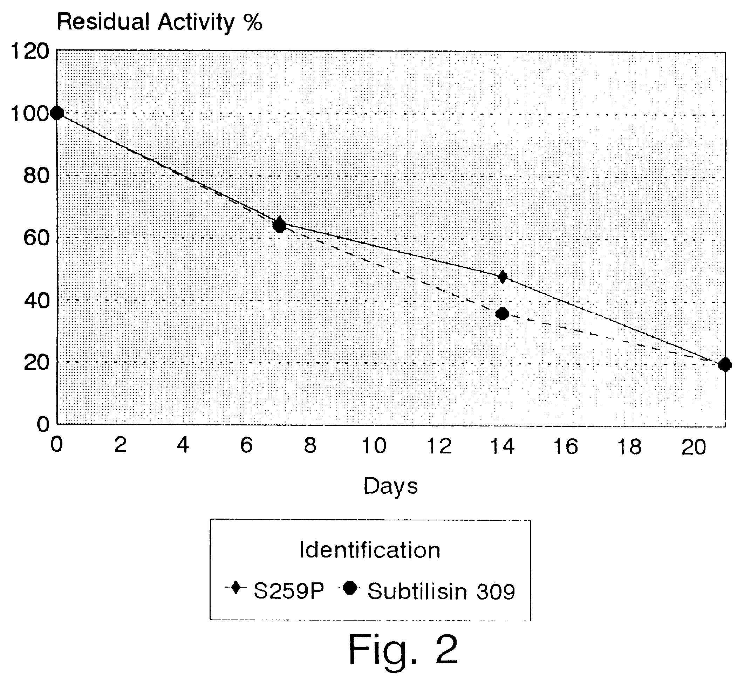

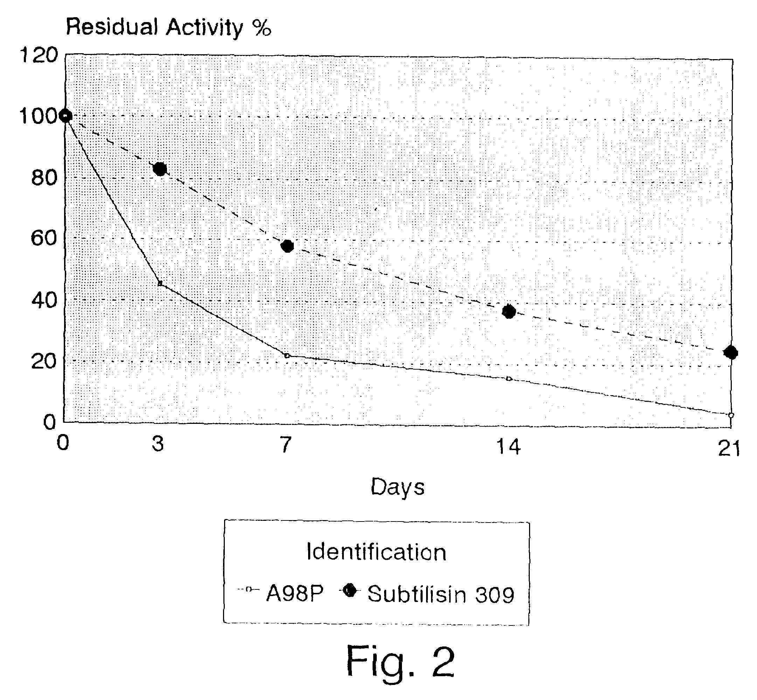

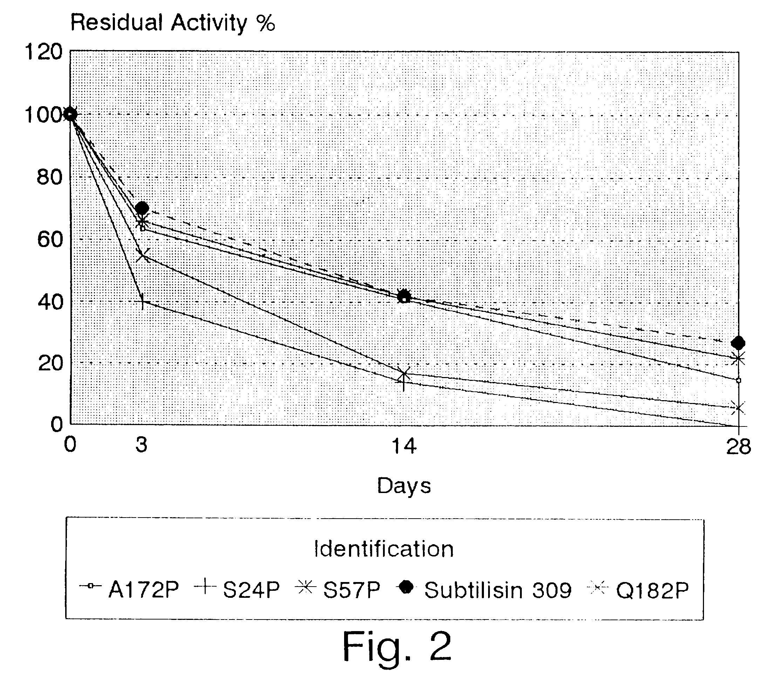

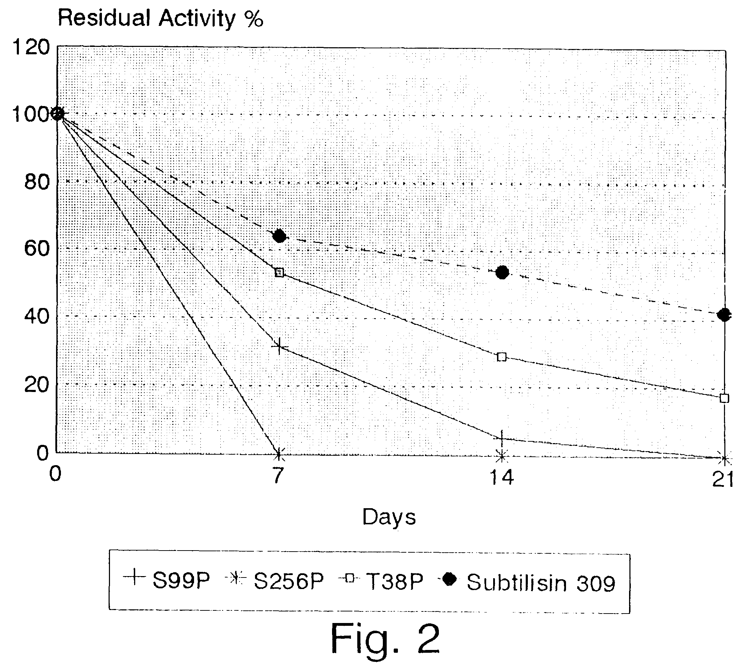

The storage stability generally reflects the thermostability, i.e. improved thermostability corresponds with improved storage stability. The improvement in thermostability of purified variants has been tested by a differential scanning calorimetry (DSC) method, *vide* Example 3 for experimental data. The result of these tests are shown in Table 3, below. The storage stability has been tested by a Mini Storage Test, *vide* Example 4 for experimental data, and the results of the storage stability test is shown in Fig. 2.

It appears from Table 3 that 3 of the variants constructed possess significantly improved thermostability, 2 of the variants possesses only slightly improved thermostability, 3 of the variants possess no significant change in thermostability, and 1 of the variants possess significantly decreased thermostability, when compared to the wild-type enzyme.

**TABLE 3**

| Stabilization relative to Subtilisin 309 | |
|---|---|
| Variant | Relative stabilization Δ DSC (°C) |
| S24P | -2.0 °C |
| T38P | -0.1 °C |
| S57P | 0.7 °C |
| A98P | 0.1 °C |
| S99P | -0.2 °C |
| A172P | 0.1 °C |
| Q182P | -2.5 °C |
| S188P | 1.5 °C |
| A194P | 2.6 °C |
| S242P | 1.4 °C |
| S256P | -2.1 °C |
| S259P | 0.6 °C |

These results demonstrate that although a clear rationale exists for stabilization by introduction of proline residues into a protein by the Phi-Psi-Concept described in this specification, no conclusion as to the stabilizing effect of the individual variants are predictable.

However, when a variant with improved thermostability has been obtained, the stabilizing effect of individual mutations in general are considered additive. This is demonstrated in Table 4 below, where the thermal denaturation temperatures, as measured by differential scanning calorimetry (DSC), of Subtilisin 309 variants relative to the wild-type enzyme are presented.

Thus stabilized protease variants which, further to the stabilizing proline residue(s) inserted, contains one or more additional stabilizing mutations as described in this specification, are considered within the scope of this invention.

**TABLE 4**

| Additive Stabilizing Effect in Subtilisin 309 Variants | | | | | | | | | |
|---|---|---|---|---|---|---|---|---|---|
| Figures in parenthesis indicate the sum of Δ DSC temperatures for variants tested individually. | | | | | | | | | |
| Variants | | | | | | | | Δ DSC (°C) | |
| G195E | | | | | | | | 0.1 | |
| | H120D | | | | | | | 0.2 | |
| | | K235L | | | | | | 0.0 | |
| | | | *36D | | | | | 4.0 | |
| | | | | N76D | | | | 4.3 | |
| | | | | | A194P | | | 2.6 | |
| | | | | | | G97N | | 0.3 | |
| | | | | | | | V104Y | 2.3 | |
| G195E | H120D | K235L | | | | | | 0.3 | (0.3) |
| G195E | H120D | K235L | *36D | | | | | 4.0 | (4.3) |
| G195E | H120D | K235L | *36D | | A194P | | | 7.0 | (6.9) |
| G195E | H120D | K235L | *36D | N76D | | | | 7.0 | (8.6) |
| G195E | H120D | K235L | *36D | N76D | A194P | | | 10.4 | (11.2) |
| G195E | H120D | | *36D | | A194P | G97N | | 7.4 | (6.6) |
| G195E | H120D | | *36D | | A194P | G97N | V104Y | 8.2 | (8.2) |
| G195E | H120D | K235L | *36D | | A194P | G97N | | 6.9 | (6.6) |
| G195E | H120D | K235L | *36D | | A194P | G97N | V104Y | 8.0 | (8.9) |
| | | | | | | | | | |
| | | | | | | | | | |

### Method For Producing Mutations In Genes Encoding Proteases

Many methods for introducing mutations into genes are well known in the art. After a brief discussion of cloning subtilisin genes, methods for generating mutations at specific sites within the subtilisin gene will be discussed.

### Cloning A Subtilisin Gene

The gene encoding subtilisin may be cloned from any Gram-positive bacteria or fungus by various methods, well known in the art. First a genomic, and/or cDNA library of DNA must be constructed using chromosomal DNA or messenger RNA from the organism that produces the subtilisin to be studied. Then, if the amino-acid sequence of the subtilisin is known, homologous, oligonucleotide probes may be synthesized, labelled, and used to identify subtilisin-encoding clones from a genomic library of bacterial DNA, or from a fungal cDNA library. Alternatively, a labelled oligonucleotide probe containing sequences homologous to subtilisin from another strain of bacteria or fungus could be used as a probe to identify subtilisin-encoding clones, using hybridization and washing conditions of lower stringency.

Yet another method for identifying subtilisin-producing clones would involve inserting fragments of genomic DNA into an expression vector, such as a plasmid, transforming protease-negative bacteria with the resulting genomic DNA library, and then plating the transformed bacteria onto agar containing a substrate for subtilisin, such as skim-milk. Those bacteria containing subtilisin-bearing plasmid will produce colonies surrounded by a halo of clear agar, due to digestion of the skim-milk by excreted subtilisin.

### Generation Of Site Directed Mutations In The Subtilisin Gene

Once the subtilisin gene has been cloned, and desirable sites for mutagenesis identified, the mutations can be introduced using synthetic oligonucleotides. These oligonucleotides contain nucleotide sequences flanking the desired mutation sites, mutant nucleotides are inserted during oligonucleotide synthesis. In a preferred method, a single stranded gap of DNA, bridging the subtilisin gene, is created in a vector bearing the subtilisin gene. Then the synthetic nucleotide, bearing the desired mutation, is annealed to a homologous portion of the single-stranded DNA. The remaining gap is then filled in by DNA polymerase I (Klenow fragment) and the construct is ligated using T4 ligase. A specific example of this method is described [*Morinaga et al*. (1984); Biotechnology, **2** 646-639]. According to *Morinaga et al*. a fragment within the gene is removed using restriction endonuclease. The vector/gene, now containing a gap, is then denatured and hybridized to a vector/gene which, instead of containing a gap, has been cleaved with another restriction endonuclease at a site outside the area involved in the gap. A single-stranded region of the gene is then available for hybridization with mutated oligonucleotides, the remaining gap is filled in by the Klenow fragment of DNA polymerase I, the insertions are ligated with T4 DNA ligase, and, after one cycle of replication, a double-stranded plasmid bearing the desired mutation is produced. The Morinaga method obviates the additional manipulation of constructing new restriction sites, and therefore facilitates the generation of mutations at multiple sites. US-A-4 760 025, by *Estell et al*., issued July 26, 1988, is able to introduce oligonucleotides bearing multiple mutations by performing minor alterations of the cassette, however, an even greater variety of mutations can be introduced at any one time by the Morinaga method, because a multitude of oligonucleotides, of various lengths, can be introduced.

### Expression Of Subtilisin Variants

According to the invention, a mutated subtilisin gene produced by methods described above, by the method described in Example 1, or any alternative methods known in the art, can be expressed, in enzyme form, using an expression vector. An expression vector generally falls under the definition of a cloning vector, since an expression vector usually includes the components of a typical cloning vector, namely, an element that permits autonomous replication of the vector in a microorganism independent of the genome of the microorganism, and one or more phenotypic markers for selection purposes. An expression vector includes control sequences encoding a promoter, operator, ribosome binding site, translation initiation signal, and, optionally, a repressor gene or various activator genes.

To permit the secretion of the expressed protein, nucleotides encoding a "signal sequence" may be inserted prior to the coding sequence of the gene. For expression under the direction of control sequences, a target gene to be treated according to the invention is operably linked to the control sequences in the proper reading frame. Promoter sequences that can be incorporated into plasmid vectors, and which can support the transcription of the mutant subtilisin gene, include but are not limited to the prokaryotic β-lactamase promoter [*Villa-Kamaroff, et al*. (1978); Proc. Natl. Acad. Sci. U.S.A.; **75** 3727-3731] and the tac promoter [*DeBoer, et al*. (1983); Proc. Natl. Acad. Sci. U.S.A.; **80** 21-25]. Further references can also be found in "Useful proteins from recombinant bacteria" in Scientific American (1980); **242** 74-94.

According to one embodiment *B. subtilis* is transformed by an expression vector carrying the mutated DNA. If expression is to take place in a secreting microorganism such as *B. subtilis* a signal sequence may follow the translation initiation signal and precede the DNA sequence of interest. The signal sequence acts to transport the expression product to the cell wall where it is cleaved from the product upon secretion. The term "control sequences" as defined above is intended to include a signal sequence, when it is present.

The microorganisms able to produce a stabilized enzyme of this invention can be cultivated by conventional fermentation methods in a nutrient medium containing assimilable carbon and nitrogen together with other essential nutrients, the medipm being composed in accordance with the principles of the known art.

### Detergent Compositions

The present invention also comprises the use of the stabilized proteases of the invention in cleaning and detergent compositions and such composition comprising the stabilized proteases.

Such compositions comprise any one or more of the proteases of the invention alone or in combination with any of the usual components included in such compositions which are well-known to the person skilled in the art.

Such components comprise builders, such as phosphate or zeolite builders, surfactants, such as anionic, cationic, non-ionic or zwitterionic type surfactants, polymers, such as acrylic or equivalent polymers, bleach systems, such as perborate- or amino-containing bleach precursors or activators, structurants, such as silicate structurants, alkali or acid to adjust pH, humectants, and/or neutral inorganic salts.

The detergent compositions of the invention can be formulated in any convenient form, such as powders, liquids, etc.

The enzymes can be used in well-known standard amounts in detergent compositions. The amounts may range very widely, e.g. about 0.0002-0.01, e.g. about 0.005-0.05, Anson units per gram of the detergent composition. Expressed in alternative units, the protease can be included in the compositions in amounts in the order of from about 0.1 to 100 GU/mg (e.g. 1-50, especially 5-20 GU/mg) of the detergent formulation, or any amount in a wide range centring at about 0.01-4, e.g. 0.1-0.4 KNPU per g detergent formulation.

The KNPU has been defined previously. A GU is a Glycine Unit, defined as the proteolytic enzyme activity which, under standard conditions, during a 15-minute incubation at 40°C, with N-acetyl casein as substrate, produces an amount of NH₂-group equivalent to 1 micromole of glycine.

It can for example be suitable to use the present enzymes at the rate of about 0.25 mg enzyme protein per litre of wash liquor, corresponding to an enzyme activity of the order of 0.08 KNPU per litre. Corresponding detergent formulations can contain the enzymes in for example an amount of the order of 0.1-0.4 KNPU/g.

The detergent compositions can also contain further enzymes.

For example, lipase can usefully be added in the form of a granular composition (alternatively a solution or a slurry) of lipolytic enzyme with carrier material (e.g. as in EP-A-258 068 (Novo Nordisk A/S) and the Lipolase™ and other enzyme compositions of Novo Nordisk A/S).

The added amount of lipase can be chosen within wide limits, for example 50 to 30,000 LU/g per gram of the surfactant system or of the detergent composition, e.g. often at least 100 LU/g, very usefully at least 500 LU/g, sometimes preferably above 1000, above 2000 LU/g or above 4000 LU/g or more, thus very often within the range of 50-4000 LU/g, and possibly within the range of 200-1000 LU/g. In this specification, lipase units are defined as they are in EP-A-258 068.

The lipolytic enzyme can be chosen from among a wide range of lipases. In particular the lipases described in for example the following patent specifications: EP-A-214 761 (Novo Nordisk A/S), EP-A-258 068, and especially lipases showing immunological crossreactivity with antisera raised against lipase from Thermomyces lanuginosus ATCC 22070, EP-A-205 208 and EP-A-206 390, and especially lipases showing immunological cross-reactivity with antisera raised against lipase from Chromobacter viscosum var lipolyticum NRRL B-3673, or against lipase from Alcaligenes PL-679, ATCC 31371 and FERM-P 3783, also the lipases described in specifications WO 87/00859 (Gist-Brocades) and EP-A-204 284 (Sapporo Breweries). Suitable in particular are for example the following commercially available lipase preparations: Lipolase Novo Nordisk A/S, Amano lipases CE, P, B, AP, M-AP, AML, and CES, and Meito lipases MY-30, OF, and PL, also Esterase MM, Lipozym, SP225, SP285, Saiken lipase, Enzeco lipase, Toyo Jozo lipase and Diosynth lipase (Trade Marks).

Amylase can for example be used when desired, in an amount in the range of about 1 to about 100 MU (maltose units) per gram of detergent composition, (or 0.014-1.4, e.g. 0.07-0.7, KNU/g (Novo units)). Cellulase can for example be used when desired, in an amount in the range of about 0.3 to about 35 CEVU units per gram of the detergent composition.

Among the usual detergent ingredients which may be present in usual amounts in the detergent compositions of this invention, are the following: The compositions may be built or unbuilt, and may be of the zero-P type (i.e. not containing any phosphorus containing builders). Thus, the composition may contain in aggregate for example from 1-50%, e.g. at least about 5% and often up to about 35-40% by weight, of one or more organic and/or inorganic builders. Typical examples of builders include those already mentioned above, and more broadly include alkali metal ortho, pyro, and tripolyphosphates, alkali metal carbonates, either alone or in admixture with calcite, alkali metal citrates, alkali metal nitrilotriacetates, carboxymethyloxysuccinates, zeolites, polyacetalcarboxylates, and so on.

Furthermore, the detergent compositions may contain from 1-35% of a bleaching agent or a bleach precursor or a system comprising bleaching agent and/or precursor with activator therefor. Further optional ingredients are lather boosters, foam depressors, anti-corrosion agents, soil-suspending agents, sequestering agents, anti-soil redeposition agents, perfumes, dyes, stabilising agents for the enzymes, and so on.

The compositions can be used for the washing of textile materials, especially but without limitation cotton and polyesterbased textiles and mixtures thereof. Especially suitable are for example washing processes carried out at temperatures of about 60-65°C or lower, e.g. about 30-35°C or lower. It can be very suitable to use the compositions at a rate sufficient to provide about e.g. 0.4-0.8 g/l surfactant in the wash liquor, although it is of course possible to use lower or greater concentrations if desired. Without limitation it can for example be stated that a use-rate from about 1 to 10 g/l, e.g. from about 3-6 g/l, of the detergent formulation is suitable for use in the case when the formulations are substantially as in the Examples.

In some useful embodiments the detergent compositions can be formulated as follows:

### Detergent I:

A detergent powder according to an embodiment of the invention containing zeolite builder is formulated to contain:

Total active detergent about 16%, anionic detergent about 9%, nonionic detergent about 6%, zeolite-containing builder about 20%, acrylic or equivalent polymer about 3.5%, perborate bleach precursor about 6-18%, amino-containing bleach activator about 2%, silicate or other structurant about 3.5%, alternatively down to about 2.5%, enzyme of about 8 (alternatively about 15) glycine units/mg grade, with alkali to adjust to desired pH in use, and neutral inorganic salt, and enzymes (about 0.5% each enzyme).

The anionic detergent is a mixture of sodium dodecyl-- benzene sulphonate, alternatively sodium linear alkyl-benzene-sulphonate, 6% and primary alkyl sulphate 3%. The nonionic detergent is an ethoxylate of an approx. C13-C15 primary alcohol with 7 ethoxylate residues per mole. The zeolite builder is type A zeolite. The polymer is polyacrylic acid. The perborate bleach precursor is sodium tetraborate tetrahydrate or monohydrate. The activator is tetraacetyl-ethylenediamine. The structurant is sodium silicate. The neutral inorganic salt is sodium sulphate.

### Detergent II:

An aqueous detergent liquid according to an embodiment of the invention is formulated to contain:

Dodecylbenzene-sulphonic acid 16%, C12-C15 linear alcohol condensed with 7 mol/mol ethylene oxide 7%, monoethanolamine 2%, citric acid 6.5%, sodium xylenesulphonate 6%, sodium hydroxide about 4.1%, protease 0.5%, minors and water to 100%. The pH is adjusted to a value between 9 and 10.

In other useful embodiments the detergent compositions can be formulated as e.g. in International Patent Publication Nos. WO 91/00334, WO 91/00335, and International Patent Application No. PCT/DK91/00399.

The invention is further illustrated in the following examples, which are not intended to be in any way limiting to the scope of the invention as claimed.

### EXAMPLE 1

### Preparation Example

In the following Example, showing a presently preferred method for constructing and expressing genes to code for wild-type and variant protease enzymes in accordance with embodiments of the present invention, the following materials are referred to:

*B. subtilis* 309 and 147 are variants of *Bacillus lentus,* deposited with the NCIB and accorded the accession numbers NCIB 10147 and NCIB 10309, and described in US-A-3 723 250 incorporated by reference herein.

*E. coli* MC 1000 *(M.J. Casadaban and S.N. Cohen* (1980); J. Mol. Biol.; **138** 179-207), was made r-,m+ by conventional methods and is also described in US-A-5 036 002.

A vector suited to a synthetic gene coding for subtilisin 309 and its mutants was constructed. It is essentially a pUC19 plasmid *[C. Yanish-Perron and J. Messing* (1985); Gene; **33** 103-119], in which the multiple cloning site has been replaced by a linker containing the restriction sites used to separate the five sub-fragments constituting the gene. The new linker was inserted into Eco RI - HindIII cut pUC19 thereby destroying these sites.

(RI) KpnI  PstI EcoRI Hind3  ClaI   SphI  Bam  (H3)
5'-AATTGGTACCCTGCAGGAATTCAAGCTTATCGATGGCATGCGGATCC-3'
3'-CCATGGGACGTCCTTAAGTTCGAATAGCTACCGTACGCCTAGGTCGA-5'

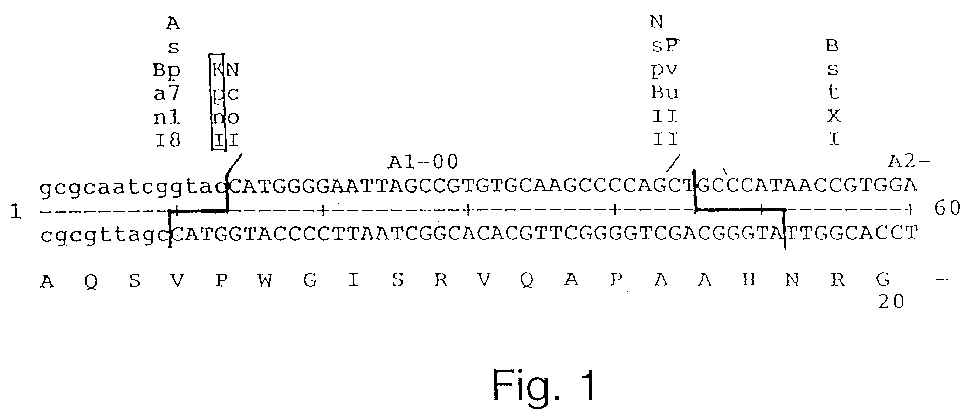

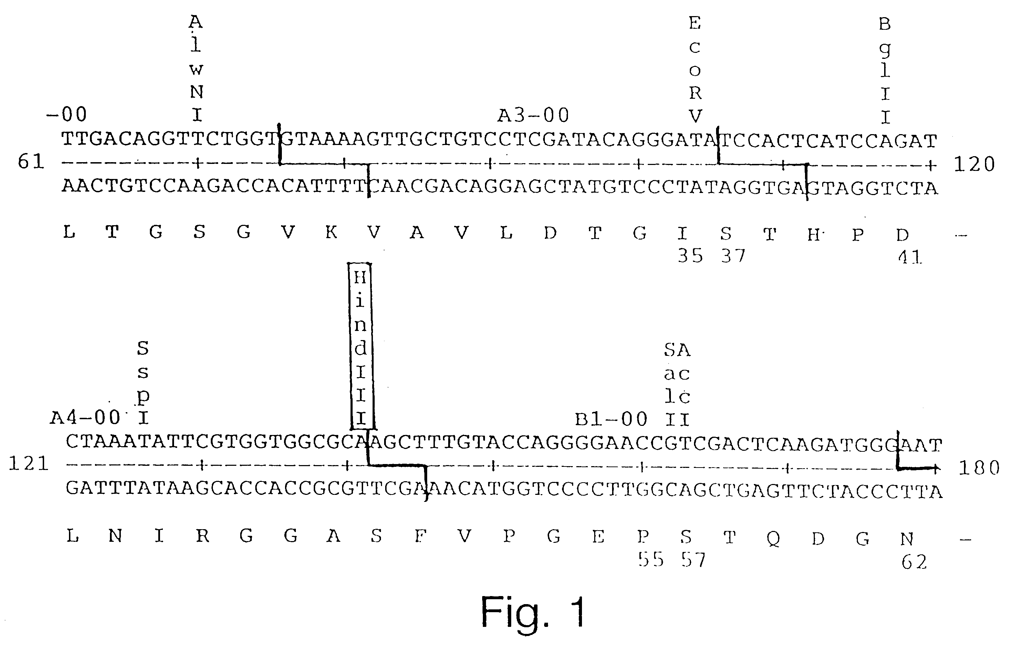

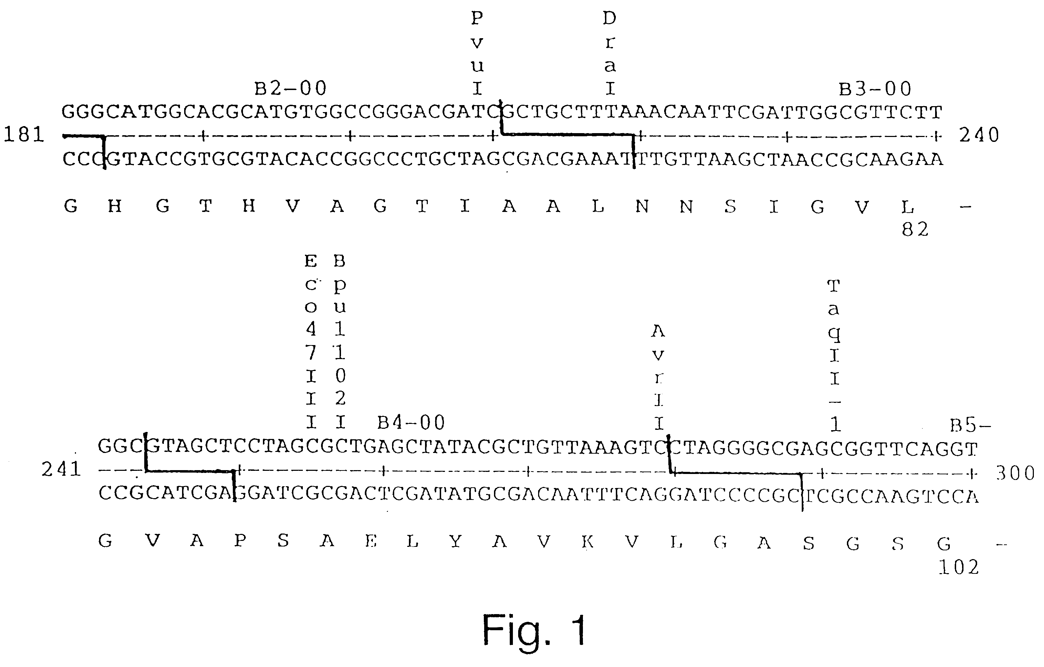

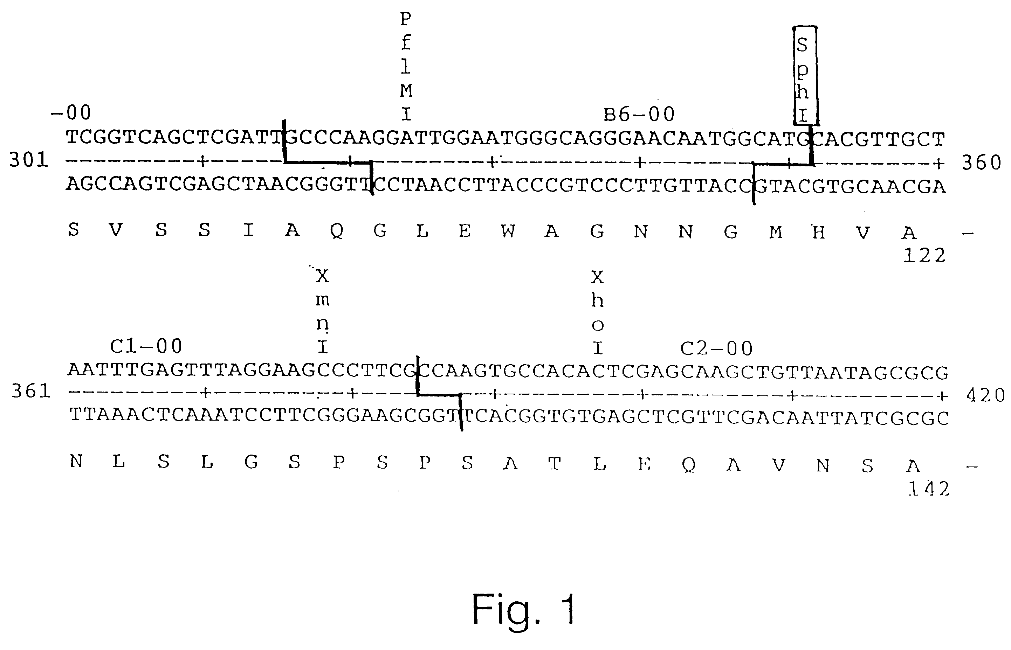

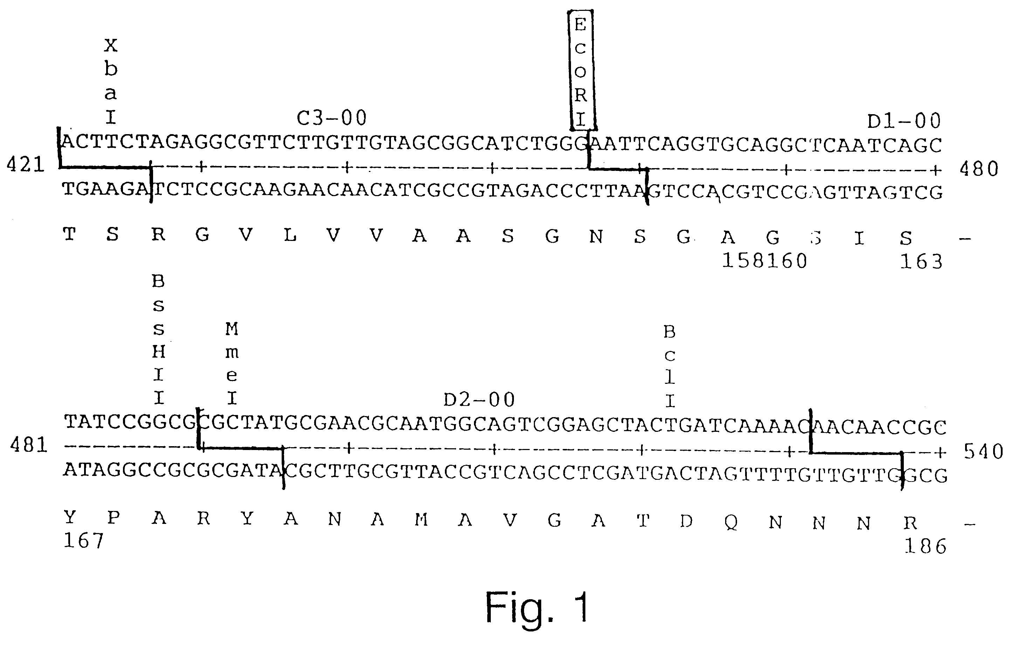

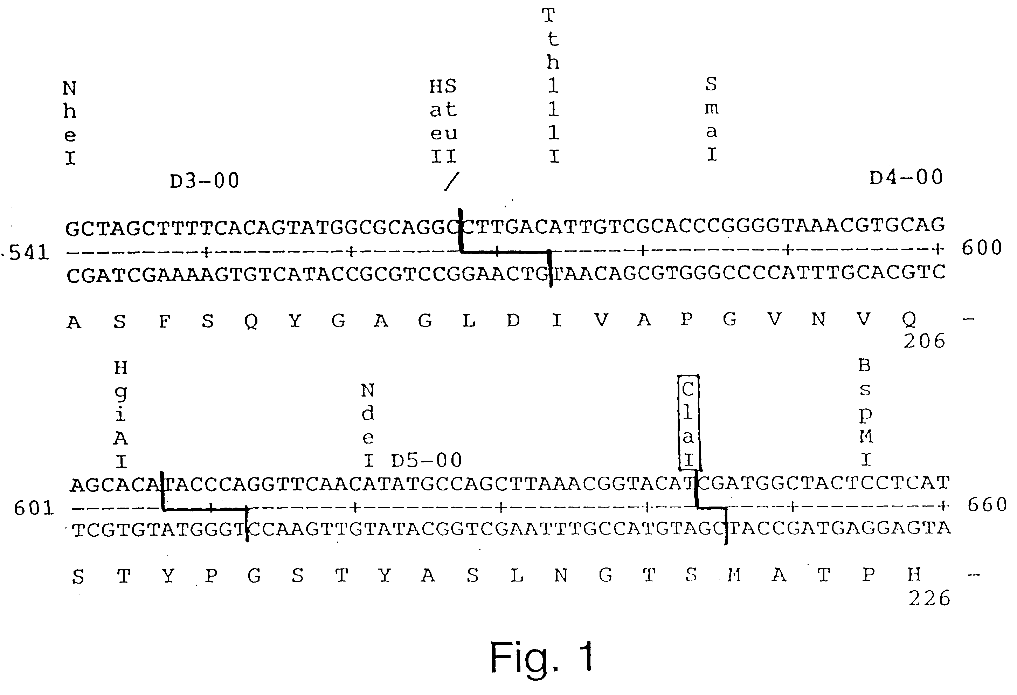

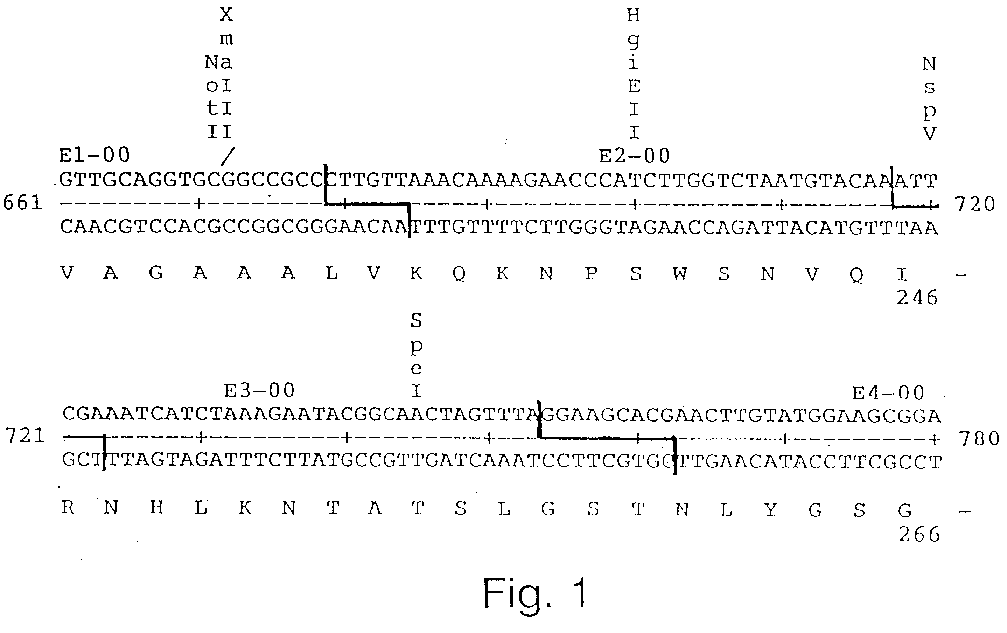

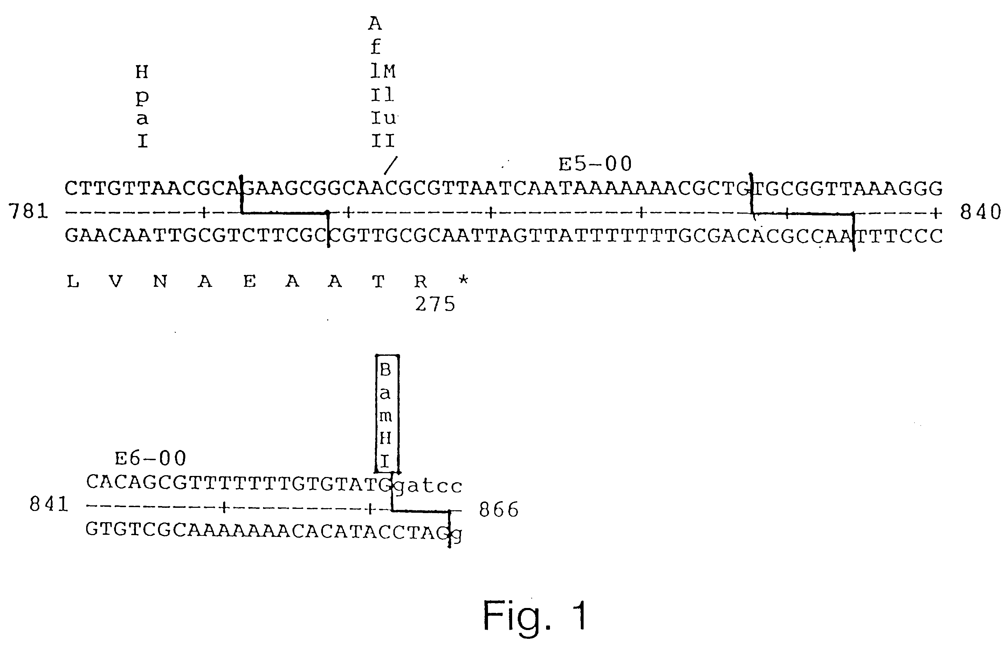

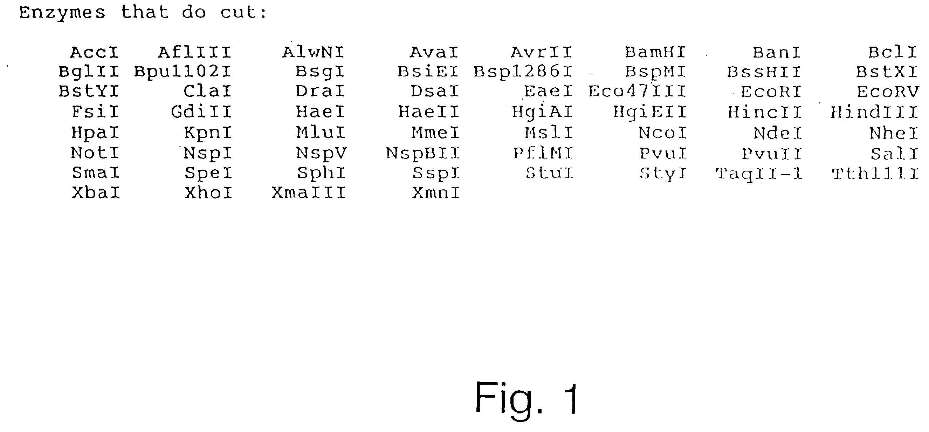

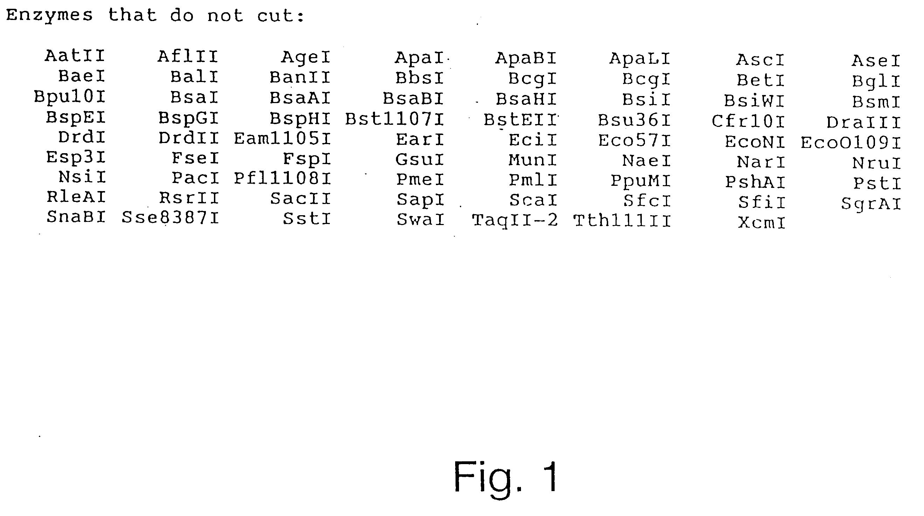

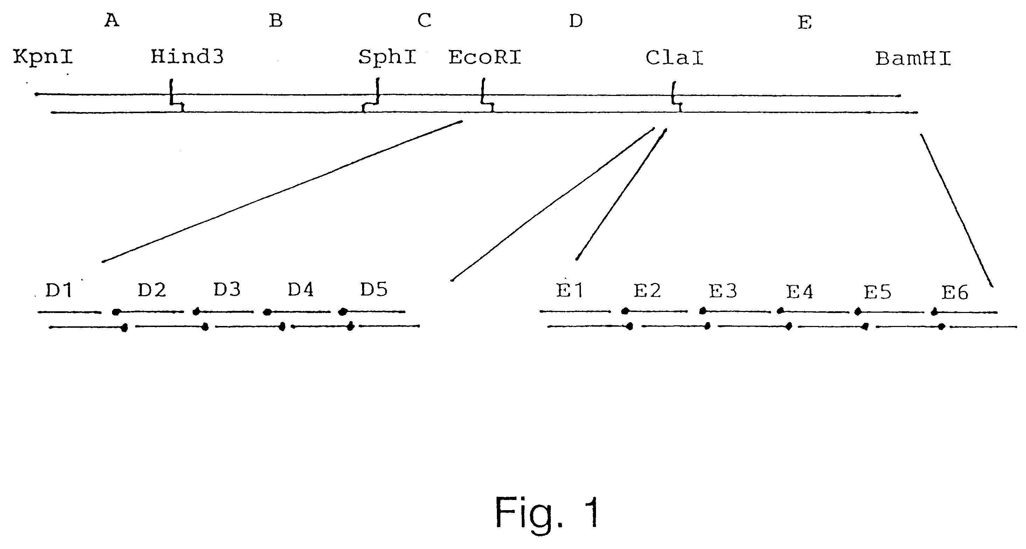

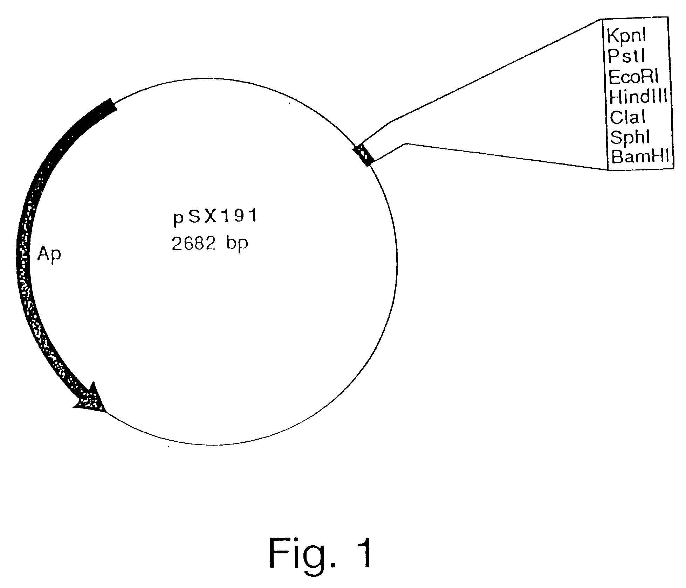

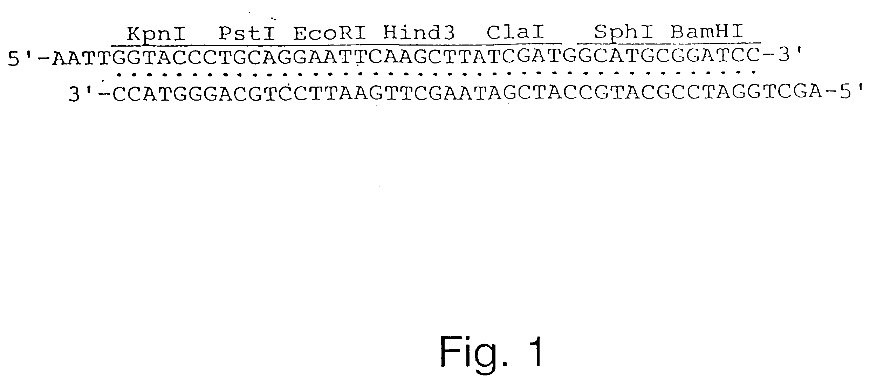

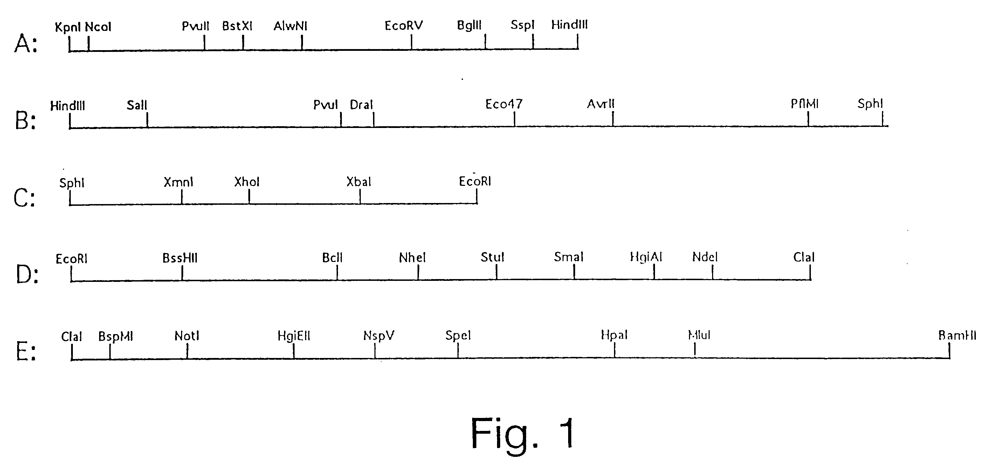

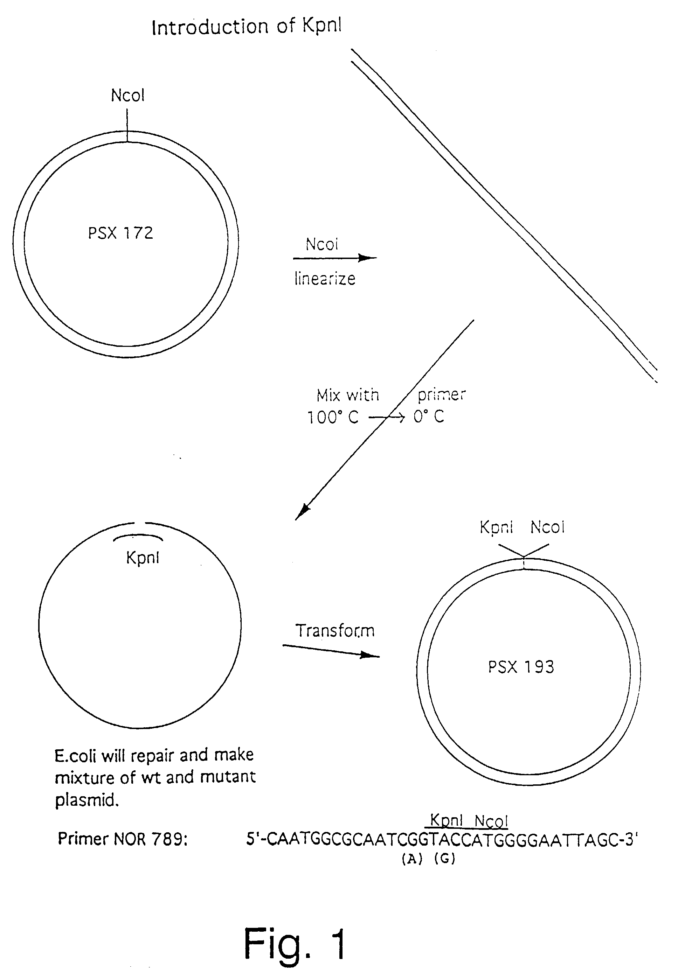

A synthetic gene coding for the mature part of subtilisin 309 was constructed as shown by the following description and the diagrams given in Figure 1 (sheets 1/7 to 7/7) of the accompanying drawings. The structure of the synthetic gene is summarised in sheets 1/7 to 4/7, which also indicate fragments used in the construction. Each subfragment was made from 6 to 12 oligonucleotides. The oligonucleotides were synthesised on an automatic DNA synthesizer using phosphoramidite chemistry on a controlled glass support [*S.L. Beaucage and M.H. Carruthers* (1981); Tetrahedron Letters; **22** 1859-1869]. Dots in the 5'-end of the oligonucleotides in the Figures are meant to indicate that these oligonucleotides have been phosphorylated. Duplexes (indicated in sheets 1/7 to 4/7) were formed from corresponding pairs of oligonucleotides by heating for 5 min at 90 deg C followed by cooling to room temperature over a period of 75 min. The duplexes were mixed and treated with T4 DNA ligase.

The five subfragments were isolated on a 2% agarose gel and inserted into pSX191. The sequence was verified by dideoxynucleotide sequencing. Fragments A-E were isolated and ligated together with KpnI-BamHI cut pSX191. The ligation mixtures were used to transform competent E coli MC1000 r-,m+ selecting for ampicillin resistance. The 850 bp KpnI-BamHI fragment that constitutes the part of the subtilisin 309 gene coding for the mature part of the enzyme was then used to replace the wild type gene on pSX212 giving rise to pSX222, which was then transformed into competent B subtilis SHa273. After fermentation of the transformed strain and purification of the enzyme it was shown that the product was indistinguishable from the wild type product.

Protease variants derived from the synthetic gene are made by using oligonucleotides with altered sequence at the place(s) where mutation is wanted (e.g. with sequences as given below) and mixing them with the rest of the oligonucleotides appropriate to the synthetic gene. Assembly of the variant gene is carried out with the variant materials in a manner otherwise analogous to that described above. Further information on synthetic genes generally is available in *Agarval et a*l (1970); Nature; **227** 27-34.

A KpnI site was introduced into the beginning of the subtilisin 309 synthetic gene encoding the mature part of the enzyme. The method used is called oligonucleotide directed double-strand break repair mutagenesis and is described by *Wlodek Mandecki* (1986); Proc. Nat. Acad. Sci. USA; **83** 7177-7181. pSX172 is opened with NcoI at the beginning of the mature part of the subtilisin 309 gene and is mixed with the oligonucleotide NOR 789 (sequence shown in Fig. 1 (7/7)), heated to 100 °C, cooled to 0 °C, and transformed into E coli. After retransformation, the recombinants can be screened by colony hybridisation using 32-P-labelled NOR 789. The recombinants that turned out to be positive during the screening had the KpnI site introduced right in front of NcoI by changing two bases without changing the amino acid sequence. pSX172 is described in EP-A-405 901. The KpnI site so created is inserted into pSX120 on a 400-bp PvuI - NheI fragment, giving rise to pSX212. pSX120 is also described in EP-A-405 901.

The synthetic gene is inserted between KpnI and BamHI on pSX212, giving rise to pSX222.

Examples of mutations and corresponding sequences of oligonucleotides are as follows:

### A194P (fragment D3)

5'- AACAACCGCGCTAGCTTTTCACAGTATGGCCCAGGC - 3'
||||||||||||||||||||||||*|||||
3'- GCGCGATCGAAAAGTGTCATACCGGGTCCGGAACTG - 5'

### A172P (fragment D2)

5'- CGCTATCCGAACGCAATGGCAGTCGGAGCTACTGATCAAAAC - 3'
      *|||||||||||||||||||||||||||||||||||
   3' - GGCTTGCGTTACCGTCAGCCTCGATGACTAGTTTTGTTGTTG - 5'

### S57P (fragment B1)

5' - AGCTTTGTACCAGGGGAACCGCCGACTCAAGATGGG - 3'
||||||||||||||||||*|||||||||||||||
3' - AACATGGTCCCCTTGGCGGCTGAGTTCTACCCTTACCC - 5'

These oligonucleotides were combined with the rest of the oligonucleotides from the synthetic gene that were not changed.

### EXAMPLE 2

### Purification Example

This procedure relates to purification of a 10 litre scale fermentation of the Subtilisin 147 enzyme, the Subtilisin 309 enzyme or mutants thereof.

Approximately 8 litres of fermentation broth were centrifuged at 5000 rpm for 35 minutes in 1 litre beakers. The supernatants were adjusted to pH 6.5 using 10% acetic acid and filtered on Seitz Supra S100 filter plates.

The filtrates were concentrated to approximately 400 ml using an Amicon CH2A UF unit equipped with an Amicon S1Y10 UF cartridge. The UF concentrate was centrifuged and filtered prior to absorption at room temperature on a Bacitracin affinity column at pH 7. The protease was eluted from the Bacitracin column at room temperature using 25% 2-propanol and 1 M sodium chloride in a buffer solution with 0.01 dimethylglutaric acid, 0.1 M boric acid and 0.002 M calcium chloride adjusted to pH 7.

The fractions with protease activity from the Bacitracin purification step were combined and applied to a 750 ml Sephadex G25 column (5 cm dia.) equilibrated with a buffer containing 0.01 dimethylglutaric acid, 0.2 M boric acid and 0.002 m calcium chloride adjusted to pH 6.5.

Fractions with proteolytic activity from the Sephadex G25 column were combined and applied to a 150 ml CM Sepharose CL 6B cation exchange column (5 cm dia.) equilibrated with a buffer containing 0.01 M dimethylglutaric acid, 0.2 M boric acid, and 0.002 M calcium chloride adjusted to pH 6.5.

The protease was eluted using a linear gradient of 0-0.1 M sodium chloride in 2 litres of the same buffer (0-0.2 M sodium chloride in case of sub 147).

In a final purification step protease containing fractions from the CM Sepharose column were combined and concentrated in an Amicon ultrafiltration cell equipped with a GR81PP membrane (from the Danish Sugar Factories Inc.).

### EXAMPLE 3

### Differential Scanning Calorimetry

The purified protease variants were subjected to thermal analysis by Differential Scanning Calorimetry (DSC).

The instrument was a Setaram micro DSC apparatus connected to HP86 computer for data collection and analysis. Setaram software was used.

The enzyme was diluted to a concentration of preferably 2 mg/ml in a liquid built detergent (pH 8.5) of the following composition:

| | |
|---|---|
| AE¹ (C₁₂₋₁₄); EO 6 | 15 % |
| LAS² (C₁₂) | 10 % |
| Coconut fatty acid | 9 % |
| Oleic acid (C₁₈) | 1 % |
| Triethanolamin (pKA 7.9) | 9 % |
| Glycerol | 10.5 % |
| Ethanol | 1.5 % |
| Sodium citrate | 8 % |
| CaCl; 2H₂O | 0.1 % |
| NaOH | 1 % |
| Water | 34.9 % |

| | |
|---|---|
| ¹) Alcohol ethoxylate | |
| ²) Linear alkylbenzene sulphonate | |

The heating rate was 0.5°C/min from 25°C to 90°C.

The stabilization of Subtilisin 309 variants relative to wild type enzyme measured by this method are presented in Table 3, above.

### EXAMPLE 4

### Storage Stability

The storage stability of three enzymes of the invention (PS1, PS3 and PS4) was determined and compared to the storage stability of subtilisin 309 (wild-type). This stability test is performed as a Mini Storage Test. In each tube 100 µl of sample were used.

The enzyme dosages were 0.25 mg enzyme/g detergent. A liquid detergent composition was used in this test, *vide* Detergent II, *supra.*

The tubes were incubated at 35°C for 3, 7, 14, and 21 days, respectively, and the residual activity determined.

The residual activity determination method was based on the digestion of a dimethyl casein (DMC) solution by the proteolytic enzymes. The primary amino groups formed in this process react with trinitrobenzene sulphonic acid (TNBS) forming a coloured complex. The reaction is followed in situ in order that the change in absorbance per time unit can be calculated.

Since the detergent might contain compounds with primary amino groups, it is necessary to examine this and make correction of the detergent effect. Correction is made by measuring the blind value of the pure detergent and subtracting this value from the value measured as described above.

The activity is determined relative to a sample which immediately after preparation is frozen and kept at a temperature of -10°C until analysis. The activity of this sample is set to 100%. The activity of the corresponding samples from the storage test is determined relative to the 100% sample.

The reaction conditions were:

| | |
|---|---|
| Temperature: | 40°C |
| pH: | 8.3 |
| Wavelength: | 420 nm |
| Reaction time: | 9 min. |
| Measuring time: | 3 min. |
| Apparatus: | 〈COBAS〉 FARA II centrifugal analyzer from Roche. |

All activities were determined in duplicate.

A folder, AF 285/1 (or later editions), describing this analytical method is available upon request to Novo Nordisk A/S, Denmark, which folder is hereby included by reference.

The result of the storage stability test is shown in Fig. 2. With respect to PS1 and PS4, these enzymes are noticeably stabilized relative to subtilisin 309. In this experiment, on the other hand, no sign of a stabilizing effect with respect to PS3 is observed.

### EXAMPLE 5

### Wash Performance

The wash performance tests were accomplished on grass juice soiled cotton in a model wash at 20°C, isothermically for 10 minutes.

As detergent 5 g/l of a powder detergent was used, *vide* Detergent I, *supra*. pH was adjusted by addition of NaOH/HCl to 10.2. The water used was approximately 9°dH (German Hardness) for the tests presented in Table 5. For the tests presented in Table 6, 6°dH water was used. The textile/wash liquor ratio was 6 g textile per litre of wash liquor.

Tests were performed at enzyme concentrations of: 0, 0.025, 0.05, 0.1, 0.5, 1.0, and 2.0 mg enzyme protein/l. Two independent sets of tests were performed for each of the enzymes. The results shown in Tables 5-6 are means of these tests.

Subsequent to washing, the fabric was rinsed in running tap water and air-dried. The protease performance was determined by the change (ΔR) of the remission (%R) at 460 nm measured on a Datacolor Elrephometer 2000, ΔR being the remission after wash with protease added minus the remission after wash with no protease added.

Results from the wash performance tests are presented in Tables 5-6. It is found that all the variants, except Subtilisin 309/S256P, perform at least equal to subtilisin 309 (wild-type).

**Table 5**

| Differential Remission, delta R | | | | | | |
|---|---|---|---|---|---|---|
| Enzyme dosage mg enzyme/l detergent | | | | | | |
| Variant | 0.025 | 0.05 | 0.1 | 0.5 | 1.0 | 2.0 |
| S57P | 2.4 | 4.4 | 7.4 | 16.8 | 20.0 | 22.1 |
| A172P | 2.4 | 4.4 | 7.5 | 16.9 | 20.1 | 22.3 |
| S188P | 2.2 | 4.8 | 7.5 | 16.9 | 20.9 | 21.7 |
| A194P | 2.5 | 5.3 | 7.6 | 17.5 | 20.5 | 20.8 |
| Subt.309 | 3.2 | 4.6 | 6.9 | 16.1 | 20.4 | 20.7 |
| S259P | 1.7 | 2.5 | 3.9 | 6.7 | 7.2 | 7.0 |
| Subt.309 | 0.8 | 2.2 | 3.6 | 5.8 | 6.5 | 6.6 |

**Table 6**

| Differential Remission, delta R | | | | | | |
|---|---|---|---|---|---|---|
| Enzyme dosage mg enzyme/l detergent | | | | | | |
| Variant | 0.025 | 0.05 | 0.1 | 0.5 | 1.0 | 2.0 |
| T38P | 1.9 | 3.7 | 7.0 | 14.7 | 16.9 | 16.7 |
| S99P | 1.8 | 3.1 | 5.4 | 15.4 | 16.4 | 16.9 |
| S256P | 0.7 | 1.5 | 3.0 | 9.7 | 15.2 | 16.8 |
| Subt.309 | 1.9 | 3.9 | 6.9 | 14.5 | 16.2 | 17.1 |

## Claims

1. An enzymatic detergent composition comprising a stabilized subtilisin, in which a naturally occurring amino acid residue (other than proline) has been substituted with a proline residue at one or more positions, at which position(s) the dihedral angles φ (phi) and ψ (psi) constitute values within the intervals [-90°<φ<-40° and -180°<ψ<180°], preferably within the intervals [-90°<φ<-40° and 120°<Ψ<180°] or [-90°<φ<-40° and -50°<Ψ<10°], and which position(s) are not located in regions, in which the protease is characterized by possessing α-helical or β-sheet structure, and which substitution is made at one or more of the positions: 57, 98, 172, 194, 242 and 259 (BPN' numbers).

2. An enzymatic detergent composition according to claim 1, in which the subtilisin further comprises one or more of the following substitutions: 27R, 36D, 76D, 97N, 98R, 104Y, 120D, 128G, 195E, 206C, 218S, 235L, 235R, 237R, 251E, and 263F (BPN' numbers).

3. An enzymatic detergent composition according to claim 1-2, in which the subtilisin is a stabilized subtilisin 309, a stabilized subtilisin 147, a stabilized subtilisin BPN', or a stabilized subtilisin Carlsberg.

4. An enzymatic detergent composition according to claim 1-2, comprising a subtilisin 309 in which one or more of the following substitutions have been introduced: S57P, A98P, A172P, A194P, S242P, and S259P (BPN' numbers).

5. An enzymatic detergent composition according to claim 1-3, wherein the subtilisin 309 further comprises one or more of the following substitutions: K27R, *36D, N76D, G97N, A98R, V104Y, H120D, S128G, G195E, Q206C, N218S, K235L, K235R, K237R, K251E, and Y263F (BPN' numbers).

6. An enzymatic detergent composition according to claims 1-5, comprising a subtilisin 309 having the following mutations: K27R+*36D+G97N+A98R+A194P+K235R+K237R+K251E+Y263F (BPN' numbers).

7. An enzymatic detergent composition according to claims 1-5, comprising a subtilisin 309 having the following mutations: K27R+*36D+G97N+A194P+K235R+K237R+K251E+Y263F (BPN' numbers).

8. An enzymatic detergent composition according to claims 1-5, comprising a subtilisin 309 having the following mutations: K27R+*36D+G97N+A194P+K235R+K237R+Y263F (BPN' numbers).

9. An enzymatic detergent composition according to claims 1-5, comprising a subtilisin 309 having the following mutations: *36D+N76D+H120D+A194P+G195E+K235L (BPN' numbers).

10. An enzymatic detergent composition according to claims 1-5, comprising a subtilisin 309 having the following mutations: *36D+G97N+V104Y+H120D+A194P+G195E (BPN' numbers).

11. An enzymatic detergent composition according to claims 1-5, comprising a subtilisin 309 having the following mutations: *36D+G97N+V104Y+H120D+A194P+G195E+K235L (BPN' numbers).

12. An enzymatic detergent composition according to claims 1-5, comprising a subtilisin 309 having the following mutations: *36D+G97N+H120D+A194P+G195E (BPN' numbers).

13. An enzymatic detergent composition according to claims 1-5, comprising a subtilisin 309 having the following mutations: *36D+G97N+H120D+A194P+G195E+K235L (BPN' numbers).

14. An enzymatic detergent composition according to claims 1-5, comprising a subtilisin 309 having the following mutations: *36D+V104Y+H120D+A194P+G195E (BPN' numbers).

15. An enzymatic detergent composition according to claims 1-5, comprising a subtilisin 309 having the following mutations: *36D+VI04Y+HI20D+A194P+G195E+K235L (BPN' numbers).

16. An enzymatic detergent composition according to claims 1-5, comprising a subtilisin 309 having the following mutations: *36D+HI20D+A194P+G195E (BPN' numbers).

17. An enzymatic detergent composition according to claims 1-5, comprising a subtilisin 309 having the following mutations: *36D+H120D+A194P+G195E+K235L (BPN' numbers).

18. An enzymatic detergent composition according to claims 1-5, comprising a subtilisin 309 having the following mutation: A194P (BPN' numbers).

## Patentansprüche

1. Enzymatische Waschmittel-Zusammensetzung umfassend ein stabilisiertes Subtilisin, bei dem ein natürlich vorkommender Aminosäurerest (außer Prolin) durch einen Prolinrest an einer oder mehreren Positionen ersetzt wurde, wobei an der Position/den Positionen die Flächenwinkel φ (phi) und ψ (psi) Werte innerhalb der Intervalle [-90°<phi<-40° und -180°<psi<180°], bevorzugt innerhalb der Intervalle [-90°<phi<-40° und 120°<psi<180°] oder [-90°<phi<-40° und -50°<psi<10°] bilden und wobei die Position/die Positionen nicht in Bereichen angeordnet sind, in denen die Protease dadurch gekennzeichnet ist, daß sie eine α-Helix- oder β-Faltblattstruktur besitzt und wobei die Substitution an einer oder mehreren der folgenden Positionen erfolgt: 57, 98, 172, 194, 242 und 259 (BPN'-Nummern).

2. Enzymatische Waschmittel-Zusammensetzung nach Anspruch 1, worin das Subtilisin weiterhin ein oder mehrere der folgenden Substitutionen umfaßt: 27R, 36D, 76D, 97N, 98R, 104Y, 120D, 128G, 195E, 206C, 218S, 235L, 235R, 237R, 251E und 263F (BPN'-Nummern).

3. Enzymatische Waschmittel-Zusammensetzung nach Anspruch 1 oder Anspruch 2, worin das Subtilisin ein stabilisiertes Subtilisin 309, ein stabilisiertes Subtilisin 147, ein stabilisiertes Subtilisin BPN' oder ein stabilisiertes Subtilisin Carlsberg ist.

4. Enzymatische Waschmittel-Zusammensetzung nach Anspruch 1 oder 2, umfassend ein Subtilisin 309, bei dem ein oder mehrere der folgenden Substitutionen eingeführt wurden: S57P, A98P, A172P, A194P, S242P und S259P (BPN'-Nummern).

5. Enzymatische Waschmittel-Zusammensetzung nach einem der Ansprüche 1 bis 3, worin das Subtilisin 309 weiterhin ein oder mehrere der folgenden Substitutionen umfaßt: K27R *36D, N76D, G97N, A98R, V104Y, H120D, S128G, G195E, Q206C, N218S, K235L, K235R, K237R, K251E und Y263F (BPN'-Nummern).

6. Enzymatische Waschmittel-Zusammensetzung nach einem der Ansprüche 1 bis 5, umfassend ein Subtilisin 309 mit den folgenden Mutationen:
K27R+*36D+G97N+A98R+A194P+K235R+K237R+K251E+Y263F (BPN'-Nummern).

7. Enzymatische Waschmittel-Zusammensetzung nach einem der Ansprüche 1 bis 5, umfassend ein Subtilisin 309 mit den folgenden Mutationen:
K27R+*36D+G97N+A194P+K235R+K237R+K251E+Y263F (BPN'-Nummern).

8. Enzymatische Waschmittel-Zusammensetzung nach einem der Ansprüche 1 bis 5, umfassend ein Subtilisin 309 mit den folgenden Mutationen:
K27R+*36D+G97N+A194P+K235R+K237R+Y263F (BPN'-Nummern).

9. Enzymatische Waschmittel-Zusammensetzung nach einem der Ansprüche 1 bis 5, umfassend ein Subtilisin 309 mit den folgenden Mutationen:
*36D+N76D+H120D+A194P+G195E+K235L (BPN'-Nummern).

10. Enzymatische Waschmittel-Zusammensetzung nach einem der Ansprüche 1 bis 5, umfassend ein Subtilisin 309 mit den folgenden Mutationen:
*36D+G97N+V104Y+H120D+A194P+G195E (BPN'-Nummern).

11. Enzymatische Waschmittel-Zusammensetzung nach einem der Ansprüche 1 bis 5, umfassend ein Subtilisin 309 mit den folgenden Mutationen:
*36D+G97N+V104Y+H120D+A194P+G195E+K235L (BPN'-Nummern).

12. Enzymatische Waschmittel-Zusammensetzung nach einem der Ansprüche 1 bis 5, umfassend ein Subtilisin 309 mit den folgenden Mutationen:
*36D+G97N+H120D+A194P+G195E (BPN'-Nummern).

13. Enzymatische Waschmittel-Zusammensetzung nach einem der Ansprüche 1 bis 5, umfassend ein Subtilisin 309 mit den folgenden Mutationen:
*36D+G97N+H120D+A194P+G195E+K235L (BPN'-Nummern).

14. Enzymatische Waschmittel-Zusammensetzung nach einem der Ansprüche 1 bis 5, umfassend ein Subtilisin 309 mit den folgenden Mutationen:
*36D+V104Y+H120D+A194P+G195E (BPN'-Nummern).

15. Enzymatische Waschmittel-Zusammensetzung nach einem der Ansprüche 1 bis 5, umfassend ein Subtilisin 309 mit den folgenden Mutationen:
*36D+V104Y+H120D+A194P+G195E+K235L (BPN'-Nummern).

16. Enzymatische Waschmittel-Zusammensetzung nach einem der Ansprüche 1 bis 5, umfassend ein Subtilisin 309 mit den folgenden Mutationen:
*36D+H120D+A194P+G195E (BPN'-Nummern).

17. Enzymatische Waschmittel-Zusammensetzung nach einem der Ansprüche 1 bis 5, umfassend ein Subtilisin 309 mit den folgenden Mutationen:
*36D+H120D+A194P+G195E+K235L (BPN'-Nummern).

18. Enzymatische Waschmittel-Zusammensetzung nach einem der Ansprüche 1 bis 5 umfasssend ein Subtilisin 309 mit der folgenden Mutation: A194P (BPN'-Nummern).

## Revendications

1. Composition détergente enzymatique comprenant une subtilisine stabilisée, dans laquelle un résidu acide aminé d'origine naturelle (autre que la proline) a été substitué par un résidu proline en une ou plusieurs positions, position(s) sur laquelle (lesquelles) les angles des dièdres φ (phi) et ψ (psi) possèdent des valeurs dans les intervalles [-90 < φ < -40 et -180° < ψ < 180 ], de préférence dans les intervalles [-90° < φ < -40 et 120° < ψ < 180°] ou [-90 < φ < -40° et -50° < ψ < 10°], cette (ces) position(s) n'étant pas située(s) dans des régions dans lesquelles la protéase est caractérisée en ce qu'elle possède une structure α-hélicoïdale ou β-lamellaire, et cette substitution étant faite à une ou plusieurs des positions: 57, 98, 172, 194, 242 et 259 (numérotation BPN).

2. Composition détergente enzymatique selon la revendication 1, dans laquelle la subtilisine comprend aussi une ou plusieurs des substitutions suivantes: 27R, 36D, 76D, 97N, 98R, 104Y, 120D, 128G, 195E, 206C, 218S, 235L, 235R, 237R, 251E et 263F (numérotation BPN).

3. Composition détergente enzymatique selon les revendications 1-2, dans laquelle la subtilisine est une subtilisine 309 stabilisée, une subtilisine 147 stabilisée, une subtilisine BPN stabilisée ou une subtilisine Carlsberg stabilisée.

4. Composition détergente enzymatique selon les revendications 1-2, comprenant une subtilisine 309 dans laquelle une ou plusieurs des substitutions suivantes ont été introduites: S57P, A98P, A172P, A194P, S242P et S259P (numérotation BPN).

5. Composition détergente enzymatique selon les revendications 1-3, dans laquelle la subtilisine 309 comprend, en outre, une ou plusieurs des substitutions suivantes: K27R, *36D, N76D, G97N, A98R, V104Y, H120D, S128G, G195E, Q206C, N218S, K235L, K235R, K237R, K251E et Y263F (numérotation BPN).

6. Composition détergente enzymatique selon les revendications 1-5, comprenant une subtilisine 309 présentant les mutations suivantes:
K27R+*36D+G97N+A98R+A194P+K235R+K237R+K251E+Y263F (numérotation BPN).

7. Composition détergente enzymatique selon les revendications 1-5, comprenant une subtilisine 309 présentant les mutations suivantes:
K27R+*36D+G97N+A194P+K235R+K237R+K251E+Y263F (numérotation BPN).

8. Composition détergente enzymatique selon les revendications 1-5, comprenant une subtilisine 309 présentant les mutations suivantes:
K27R+*36D+G97N+A194P+K235R+K237R+Y263F (numérotation BPN).

9. Composition détergente enzymatique selon les revendications 1-5, comprenant une subtilisine 309 présentant les mutations suivantes: *36D+N76D+H120D+A194P+G195E+K235L (numérotation BPN).

10. Composition détergente enzymatique selon les revendications 1-5, comprenant une subtilisine 309 présentant les mutations suivantes: *36D+G97N+V104Y+H120D+A194P+G195E (numérotation BPN).

11. Composition détergente enzymatique selon les revendications 1-5, comprenant une subtilisine 309 présentant les mutations suivantes: *36D+G97N+V104Y+H120D+A194P+G195E+K235L (numérotation BPN).

12. Composition détergente enzymatique selon les revendications 1-5, comprenant une subtilisine 309 présentant les mutations suivantes: *36D+G97N+H120D+A194P+G195E (numérotation BPN).

13. Composition détergente enzymatique selon les revendications 1-5, comprenant une subtilisine 309 présentant les mutations suivantes: *36D+G97N+H120D+A194P+G195E+K235L (numérotation BPN).

14. Composition détergente enzymatique selon les revendications 1-5, comprenant une subtilisine 309 présentant les mutations suivantes: *36D+V104Y+H120D+A194P+G195E (numérotation BPN).

15. Composition détergente enzymatique selon les revendications 1-5, comprenant une subtilisine 309 présentant les mutations suivantes: *36D+V104Y+H120D+A194P+G195E+K235L (numérotation BPN).

16. Composition détergente enzymatique selon les revendications 1-5, comprenant une subtilisine 309 présentant les mutations suivantes: *36D+H120D+A194P+G195E (numérotation BPN).

17. Composition détergente enzymatique selon les revendications 1-5, comprenant une subtilisine 309 présentant les mutations suivantes: *36D+H120D+A194P+G195E+K235L (numérotation BPN).

18. Composition détergente enzymatique selon les revendications 1-5, comprenant une subtilisine 309 présentant la mutation suivante: A194P (numérotation BPN).
